# EUROPÄISCHE PATENTSCHRIFT

(11) **EP 3 266 075 B1**
(45) Veröffentlichungstag und Bekanntmachung des Hinweises auf die Patenterteilung: **01.06.2022**
(21) Anmeldenummer: 16708112.4
(22) Anmeldetag: 01.03.2016
(51) Int. Cl.: H01R 13/627, A61M 5/14, A61M 5/168, A61M 39/02, A61M 39/22, A61M 39/10, A61M 39/00

(54) **MEDIZINISCHER STECK- UND RASTKONNEKTOR ZUM HERSTELLEN EINER FLUIDVERBINDUNG ZWISCHEN ZWEI SYSTEMEN**
MEDICAL PLUG-IN AND SNAP-IN CONNECTOR FOR ESTABLISHING A FLUID CONNECTION BETWEEN TWO SYSTEMS
CONNECTEUR MÉDICAL D'ENFICHAGE ET D'ENCLIQUETAGE POUR LA CRÉATION D'UNE COMMUNICATION DE FLUIDE ENTRE DEUX SYSTÈMES

(30) Priorität: 02.03.2015 DE 102015102990
(43) Veröffentlichungstag der Anmeldung: 10.01.2018
(73) Patentinhaber: Fresenius Medical Care Deutschland GmbH, 61352 Bad Homburg (DE)
(72) Erfinder: LAUER, Martin, 66606 St. Wendel (DE)
(74) Vertreter: Bobbert & Partner Patentanwälte PartmbB
(86) Internationale Anmeldenummer: PCT/EP2016/054306
(87) Internationale Veröffentlichungsnummer: WO 2016/139197

(56) Entgegenhaltungen:
- WO-A1-90/05559
- WO-A1-2014/021390
- WO-A2-2007/118235
- CN-A- 87 105 316
- GB-A- 2 091 365
- JP-A- 2010 137 043
- US-A- 5 496 274

## Beschreibung

Die vorliegende Erfindung betrifft einen Steck- und/oder Rastkonnektor gemäß Anspruch 1. Sie betrifft zudem ein Set gemäß Anspruch 8 eine Blutbehandlungsvorrichtung oder einen Verbindungsadapter gemäß Anspruch 11 eine Schlauchleitung gemäß Anspruch 12 und eine Behandlungsmaschine gemäß Anspruch 14

Aus dem Stand der Technik sind Verbinder oder Konnektoren zum Herstellen einer Fluidverbindung zwischen zwei Systemen, welche jeweils Fluid führen, bekannt. Beispiele des Stands der Technik finden sich in den Dokumenten WO 90/05559 A1, Gb 2 091 365 A, US 5 496 274 A, WO 2007/118235 A2, JP 2010 137043 A, WO 2014/021390 A1, CN 87 105 3116 A.

Eine Aufgabe der vorliegenden Erfindung ist es, einen weiteren Konnektor (auch als Verbinder bezeichnet) zum Herstellen einer Fluidverbindung zwischen zwei Systemen, welche ein medizinisches Fluid führen, vorzuschlagen. Zudem sollen geeignete Vorrichtungen, welche mit dem erfindungsgemäßen Konnektor direkt oder indirekt verbunden sind, angegeben werden.

Die erfindungsgemäße Aufgabe wird durch einen Steckkonnektor mit den Merkmalen des Anspruchs 1 gelöst. Sie wird ferner gelöst mittels des Sets mit den Merkmalen des Anspruchs 8, einer Blutbehandlungsvorrichtung oder eines Verbindungsadapters mit den Merkmalen des Anspruchs 11, einer Schlauchleitung mit den Merkmalen des Anspruchs 12 und einer Behandlungsmaschine mit den Merkmalen des Anspruchs 14.

Erfindungsgemäß wird somit ein Steck- und/oder Rastkonnektor (kurz: Steckkonnektor) vorgeschlagen, welcher ausgestaltet ist, um auf einen Konnektionsabschnitt aufgesteckt oder aufgeschoben zu werden. Der Konnektionsabschnitt kann Teil einer Blutbehandlungsvorrichtung, eines Verbindungsdapters, einer Fluidleitung oder dergleichen sein. Der Steckkonnektor kann Teil einer Fluidleitung oder Fluidverbindung sein, etwa zwischen der Blutbehandlungsvorrichtung und der Fluidleitung oder zwischen dem Verbindungsadapter und der Fluidleitung.

Vorzugsweise dient der Steckkonnektor einer Verbindung von Leitungen, in welchen medizinische Fluide wie Blut oder Dialysierflüssigkeit geführt werden. Er kann deshalb auch als medizinischer Steckkonnektor bezeichnet werden.

Der Steckkonnektor kann beispielsweise dem Verbinden einer Blutbehandlungsvorrichtung mit einer Fluidleitung dienen. Erstere kann als Blutfilter oder Dialysator ausgestaltet sein. Letztere kann als eine Blut- oder Dialysierflüssigkeitsleitung ausgestaltet sein.

Der Konnektionsabschnitt kann insbesondere als ein Schlauchverbinder ausgestaltet sein.

Der Steckkonnektor weist wenigstens eine erste Komponente auf, welche hier als Grundkörper bezeichnet wird (diese beiden Begriffe sind hierin austauschbar), in bestimmten erfinderischen Ausführungsformen allerdings auch als Griffteil oder Gehäuseteil bezeichnet werden könnte. Der Grundkörper weist einen Aufnahmeabschnitt zum Aufnehmen eines Endes eines Fluidrohrs des Konnektionsabschnitts auf. Der Grundkörper weist ferner wenigstens ein erstes Rastelement und ein optional zweites Rastelement auf.

Das erste Rastelement weist einen ersten Abschnitt auf. Dieser ist elastisch biegbar oder kippbar ausgestaltet. Nur vorzugsweise ist der erste Abschnitt an einem freien Ende des ersten Rastelements angeordnet oder schließt das erste Rastelement an dessen freien Ende ab.

Das zweite Rastelement weist einen hier als zweiten Abschnitt bezeichneten Abschnitt auf. Dieser ist rein vorzugsweise an einem Ende des zweiten Rastelements angeordnet oder schließt das zweite Rastelement an dessen freiem Ende ab. Das zweite Rastelement ist rein optional und nicht unverzichtbar für die vorliegende Erfindung.

Erfindungsgemäß weist wenigstens das erste Rastelement und/oder das zweite Rastelement eine Vertiefung oder eine Erhebung auf. Die Vertiefung, sofern vorhanden, dient der Aufnahme eines Vorsprungs des Konnektionsabschnitts, welcher wiederum Teil beispielsweise einer Blutbehandlungsvorrichtung oder eines Verbindungsadapters ist. Die Erhebung, sofern vorhanden, dient dem Eingreifen in eine Vertiefung des Konnektionsabschnitts.

Dabei sind die Vertiefung oder die Erhebung so angeordnet, dass sie dann, wenn sie Teil des ersten Rastelements ist, dem zweiten Rastelement (und/oder dem Aufnahmeabschnitt) zugewandt ist, und/oder umgekehrt. Ergänzend oder alternativ ist die Vertiefung oder die Erhebung an einer Innenseite des jeweiligen Rastelements angeordnet.

Das erfindungsgemäße Set besteht aus wenigstens einem erfindungsgemäßen Steckkonnektor und wenigstens einem Konnektionsabschnitt oder weist beide auf, wobei Steckkonnektor und Konnektionsabschnitt ausgestaltet und vorgesehen sind, um miteinander in Rastverbindung und vorzugsweise in Fluidverbindung verrastet zu werden. Letzteres geschieht vorzugsweise kraft- und/oder formschlüssig.

Die Erfindung betrifft ferner eine Blutbehandlungsvorrichtung oder einen Verbindungsadapter mit jeweils wenigstens einem Konnektionsabschnitt. Der Konnektionsabschnitt ist ausgestaltet und vorgesehen, um mit wenigstens einem erfindungsgemäßen Steckkonnektor in Rastverbindung - und vorzugsweise in Fluidverbindung - verrastet zu werden. Der Konnektionsabschnitt kann dadurch ausgestaltet und vorgesehen sein, dass er Vorsprünge oder Vertiefungen hat, welche zum Herstellen einer Rastverbindung mit einem Steckkonnektor unter Erzielen einer Fluidverbindung zwischen einem Fluidrohr des Konnektionsabschnitt und dem Fluidführungsabschnitt des Steckkonnektors geeignet, ausgestaltet und/oder vorgesehen sind.

Die erfindungsgemäße medizinische oder medizintechnische Behandlungsvorrichtung (im Folgenden auch kurz: Behandlungsvorrichtung) ist ausgestaltet, um in Verbindung mit einer Blutbehandlungsmaschine zur Dialyse, Hämodialyse, Hämodiafiltration, Filtration oder Apherase zu dienen. Sie kann als Blutfilter, Dialysator oder dergleichen ausgestaltet sein.

Die erfindungsgemäße Schlauchleitung für die medizinische Verwendung weist wenigstens einen erfindungsgemäßen Steckkonnektor auf.

Die erfindungsgemäße medizinische oder medizintechnische Behandlungsmaschine ist mit einer erfindungsgemäßen Blutbehandlungsvorrichtung in Fluidkommunikation verbunden. Die Blutbehandlungsmaschine kann der Dialyse, Hämodialyse, Hämodiafiltration, Filtration oder Apharase dienen und die hierzu erforderlichen Vorrichtungen wie Blutpumpen usw. aufweisen.

Bei allen Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Erfindungsgemäße Ausführungsformen können eines oder mehrere der vorstehend oder im Folgenden genannten Merkmale in beliebiger Kombination aufweisen. Erfindungsgemäße Ausführungsformen sind ferner Gegenstand der Unteransprüche.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Die Angaben "oben" und "unten" sind hierin bei Zweifel des Fachmanns als absolute oder relative Raumangaben zu verstehen, welche sich auf die Ausrichtung des betreffenden Bauteils während seines üblichen Gebrauchs beziehen.

In bestimmten, beispielhaften erfindungsgemäßen Ausführungsformen stellt sich die Vertiefung ein, wenn keine äußeren Kräfte auf das erste und/oder das zweite Rastelement wirken. Die Vertiefung kann somit durch Einwirken auf das erste und/oder das zweite Rastelement und ihr elastisches Verformen aufgehoben oder derart in ihrer Geometrie verändert werden, dass ein Zurückhalten des Vorsprungs nicht oder nicht in allen Verschieberichtung (in Längsrichtung des Steckkonnektors) mehr gewährleistet ist. Sie ist erneut zu beobachten, wenn das aktive, elastische Verformen beendet wird. Dies kann beispielsweise gestalterisch dadurch umgesetzt sein, dass der freie Endabschnitt des ersten und/oder des zweiten Rastelements relativ zum übrigen Rastelement oder zum Grundkörper kippbar, klappbar, bewegbar oder anderweitig, insbesondere gegen Materialrückstellkräfte oder Materialwiderstände, angeordnet ist.

In bestimmten, beispielhaften erfindungsgemäßen Ausführungsformen weist der Grundkörper einen durch ein Inneres des Grundkörpes verlaufenden Fluidführungsabschnitt auf, welcher sich in einer Längsrichtung des Grundkörpers durch diesen hindurch erstreckt. Der Fluidführungsabschnitt kann ein Fluidkanal sein.

In bestimmten, beispielhaften erfindungsgemäßen Ausführungsformen ist der Grundkörper einstückig gefertigt, in anderen mehrstückig gefertigt.

In bestimmten, beispielhaften erfindungsgemäßen Ausführungsformen sind der Vorsprung oder die Vorsprünge des Konnektionsabschnitts als Rastkante ausgestaltet. Letztere kann umlaufend sein, also den gesamten Umfang der sie tragenden Komponente, beispielsweise des Konnektionsabschnitts, umgeben, oder nur Abschnitte hiervon.

In bestimmten, beispielhaften erfindungsgemäßen Ausführungsformen ist wenigstens eines der Rastelemente als Rastflügel, Klemmflügel, Arm, Komponente, welche die Vertiefung oder die Erstreckung trägt, oder dergleichen ausgestaltet.

In manchen beispielhaften erfindungsgemäßen Ausführungsformen ist der zweite Abschnitt des zweiten Rastelements ebenfalls elastisch biegbar oder elastisch kippbar ausgestaltet. In diesen Ausführungsformen erfolgt das Verrasten unter elastischem Biegen oder Kippen beider Rastelemente. In anderen genügt ein elastisches Biegen oder Kippen nur des ersten Rastelements. Das zweite Rastelement, sofern vorhanden, dient, falls es keinen elastischen Abschnitt aufweist, als Gegenlager.

In bestimmten, beispielhaften erfindungsgemäßen Ausführungsformen sind der erste Abschnitt des ersten Rastelements einerseits und der zweite Abschnitt des zweiten Rastelements oder der Aufnahmeabschnitt andererseits in einem Nichtverbindungszustand unter einem ersten Abstand derart zueinander angeordnet, dass der erste Abstand beim Vorgang des Verbindens oder zum Zwecke des Verbindens des Steckkonnektors mit einem Konnektionsabschnitt in einen zweiten Abstand übergeht oder übergehen muss, auch wenn dies nur vorübergehend ist, welcher größer als der erste Abstand ist.

In bestimmten, beispielhaften erfindungsgemäßen Ausführungsformen liegen der erste Abstand und der zweite Abstand in derselben Ebene.

In einigen erfindungsgemäßen Ausführungsformen liegen der erste Abschnitt des ersten Rastelements und der zweite Abschnitt des zweiten Rastelements in einer Ebene oder sind in einer gemeinsamen Ebene zum Verbinden oder Lösen der Verbindung bewegbar. Dies erlaubt es vorteilhaft, die Verbindung mit nur einer Hand bequem zu lösen.

In bestimmten, beispielhaften erfindungsgemäßen Ausführungsformen sind der erste Abschnitt des ersten Rastelements und/oder der zweite Abschnitt des zweiten Rastelements als kippbarer Abschnitt ausgestaltet oder kippbar an einem weiteren Abschnitt des entsprechenden Rastelements angeordnet.

In bestimmten, beispielhaften erfindungsgemäßen Ausführungsformen sind das erste und/oder das zweite Rastelement integrale oder einstückig hergestellte Abschnitte des Grundkörpers.

In bestimmten, beispielhaften erfindungsgemäßen Ausführungsformen beträgt ein Innendurchmesser des Fluidführungsabschnitts des Grundkörpers, welcher sich insbesondere in Längsrichtung des Grundkörpers erstreckt, konstant und vorzugsweise zwischen 4,0 und 4,5 mm, besonders bevorzugt 4,2 mm.

In bestimmten, beispielhaften erfindungsgemäßen Ausführungsformen besteht der Grundkörper aus einem ersten Material oder weist ein solches auf. Zudem weist der Steckkonnektor wenigstens ein Dichtelement aus einem zweiten, vom ersten Material verschiedenen Material auf.

In bestimmten, beispielhaften erfindungsgemäßen Ausführungsformen weist das Dichtelement eine Durchgangsöffnung auf mit genau einem oder wenigstens einem Innendurchmesser, welcher einem Innendurchmesser des Fluidführungsabschnitts des Grundkörpers entspricht oder identisch zu diesem ist.

In bestimmten, beispielhaften erfindungsgemäßen Ausführungsformen weist der Grundkörper zusätzlich zum ersten Endbereich und zum zweiten Endbereich des Fluidführungsabschnitts wenigstens eine weitere Öffnung auf. Die Öffnung ist mittels Septums verschließbar und weist optional ein solches Septum auf. Diese Öffnung liegt oder mündet vorzugsweise zwischen dem ersten Endbereich und dem zweiten Endbereich in den Fluidführungsabschnitt.

In bestimmten, beispielhaften erfindungsgemäßen Ausführungsformen weist der Grundkörper den Fluidführungsabschnitt als einen Hauptkanal mit einem Lumen zum Leiten eines ersten Fluids durch den Steckkonnektor hindurch auf. Der Grundkörper weist ferner eine Nebenkanalmündung eines Nebenkanals zum Zugeben eines zweiten Fluids in den Hauptkanal auf. Dabei weist der Steckkonnektor wenigstens ein relativ zum Grundkörper aus einer ersten Position in eine zweite Position überführbar angeordnetes Betätigungselement auf. Ferner weist der Steckkonnektor einen Dichtabschnitt auf, welcher angeordnet ist, um bei Überführen des Betätigungselements von einer Position in die andere zwischen einer ersten Stellung des Dichtabschnitts, in welcher der Dichtabschnitt die Nebenkanalmündung nicht verschließt oder bedeckt (offene Stellung) und einer zweiten Stellung des Dichtabschnitts, in welcher der Dichtabschnitt die Nebenkanalmündung verschließt oder bedeckt (geschlossene Stellung) verdrehbar zu sein.

In bestimmten, beispielhaften erfindungsgemäßen Ausführungsformen des Sets sind der Innendurchmesser des Fluidführungsabschnitts und ein Innendurchmesser eines Fluidrohrs des Konnektionsabschnitts im Bereich eines dem Fluidführungsabschnitt zugeordneten oder zugewandten Stirnseite im Wesentlichen oder vollkommen gleich.

In bestimmten, beispielhaften erfindungsgemäßen Ausführungsformen des Sets sind der Innendurchmesser des Fluidführungsabschnitts, ein Innendurchmesser eines Dichtelements und ein Innendurchmesser des Fluidrohrs im Bereich der dem Fluidführungsabschnitt zugeordneten oder zugewandten Stirnseite im Wesentlichen oder vollkommen gleich.

In bestimmten, beispielhaften erfindungsgemäßen Ausführungsformen ist die Schlauchleitung maximal 200 mm (Millimeter), vorzugsweise maximal 150 mm, ganz besonders bevorzugt maximal 100 mm lang.

In manchen erfindungsgemäßen Ausführungsformen ist der Steckkonnektor kein Schraubkonnektor, weist kein Gewinde auf und/oder ist mit keiner Gewindemutter oder Überwurfmutter zu seiner Verbindung ausgestaltet oder verbindbar

In einigen erfindungsgemäßen Ausführungsformen ist der Steckkonnektor Teil einer Fluidleitung. D. h., dass ein Abschnitt des Steckkonnektors, welcher hierin als Fluidführungsabschnitt bezeichnet ist, im bestimmungsgemäßen Gebrauch von Fluid durchströmt wird oder werden kann, da dieser Abschnitt Teil eines Strömungspfads ist.

Der Schlauch, welcher mit dem Steckkonnektor in manchen erfindungsgemäßen Ausführungsformen verbunden ist, kann seinerseits mit einer Blutkassette verbunden sein. Die Blutkassette kann ein Hartteil sein, welches auf mindestens einer Seite mittels Folie gegenüber einer Umgebung abgeschlossen und/oder mit der Folie verbunden ist.

In bestimmten erfindungsgemäßen Ausführungsformen sind das erste Rastelement und/oder das zweite Rastelement als Biegegabel ausgestaltet. Das erste Rastelement und/oder das zweite Rastelement können eine oder mehrere Rastnasen aufweisen. Letztere können an der Biegegabel vorgesehen sein.

In beispielhaften erfindungsgemäßen Ausführungsformen sind die Vertiefungen wenigstens eines Rastelements an einer Innenseite oder Unterseite hiervon ausgestaltet.

In einigen erfindungsgemäßen Ausführungsformen ist der Steckkonnektor ausgestaltet, um mit dem Konnektionsabschnitt, ausschließlich mittels Verrastens des ersten Rastelements und/oder des zweiten Rastelements, und mittels Einsteckens des Fluidrohrs oder eines Stirnabschnitts hiervon in den Aufnahmeabschnitt sowie optional mittels Aufschieben eines äußeren Rohrs des Konnektionsabschnitts auf den Aufnahmeabschnitt verbunden zu werden.

In einigen beispielhaften erfindungsgemäßen Ausführungsformen ist der Hauptkanal ein Rohr.

In einigen beispielhaften erfindungsgemäßen Ausführungsformen ist der Dichtabschnitt angeordnet und ausgestaltet, um eine Durchgängigkeit des Hauptkanals für das erste Fluid weder in seiner ersten Stellung noch in seiner zweiten Stellung zu beeinträchtigen.

In einigen beispielhaften erfindungsgemäßen Ausführungsformen liegt kein Abschnitt des Dichtabschnitts im Lumen des Hauptkanals vor.

In einigen beispielhaften erfindungsgemäßen Ausführungsformen weist der Hauptkanal zusätzlich zur Nebenkanalmündung eine Septummündung auf, welche im Gebrauch des Steckkonnektors mit einem mittels Kanüle durchstechbaren Septum verschlossen ist.

In einigen beispielhaften erfindungsgemäßen Ausführungsformen münden die Nebenkanalmündung und/oder die Septummündung in einen gerade verlaufenden Abschnitt eines Querschnitts des Lumens oder dessen Umfangs des Hauptkanals.

In einigen beispielhaften erfindungsgemäßen Ausführungsformen weist der Dichtabschnitt eine stirnseitige Abdichtfläche auf, welche angeordnet ist, um bei einer Drehung des Dichtabschnitts entlang einer Drehkurve von einer ersten Stellung in eine zweite Stellung auf der Drehkurve bewegt zu werden, wobei die Abdichtfläche in der zweiten Stellung die Nebenkanalmündung verschließt oder bedeckt, wobei die Abdichtfläche die Nebenkanalmündung in der ersten Stellung nicht verschließt oder bedeckt, und wobei sich die Abdichtfläche parallel zu einer Hauptquerschnittsebene und/oder senkrecht zu einer Drehachse des Dichtabschnitts erstreckt.

In einigen beispielhaften erfindungsgemäßen Ausführungsformen weist der Dichtabschnitt eine Dichtnase auf, welche in axialer Richtung des Dichtabschnitts über diesen vorsteht, wobei die Dichtnase angeordnet ist, um bei einer Drehung des Betätigungselements, oder beim Überführen des Betätigungselements von einer Position in die andere, auf einer Drehkurve von einer ersten Stellung in eine zweite Stellung auf der Drehkurve bewegt zu werden, wobei die Dichtnase in der ersten Stellung die Nebenkanalmündung nicht verschließt oder bedeckt, und wobei die Dichtnase die Nebenkanalmündung in der zweiten Stellung verschließt oder bedeckt.

In einigen beispielhaften erfindungsgemäßen Ausführungsformen weist das Gehäuseelement wenigstens einen Abschnitt auf, welcher eine Vertiefung zur Aufnahme der hierin bewegbaren Dichtnase aufweist, und welcher die Nebenkanalmündung aufweist oder hieran angrenzt.

In einigen beispielhaften erfindungsgemäßen Ausführungsformen weist die Dichtnase eine sowohl zu einer Stirnseite der Dichtnase als auch zu einer lateralen Seitenfläche oder Umfangsfläche des Dichtabschnitts offene Nut auf.

In einigen beispielhaften erfindungsgemäßen Ausführungsformen liegt die Nut in der ersten Stellung des Dichtabschnitts derart an einer Öffnung eines Nebenkanalrohrs an, dass sie den Fluidweg des Nebenkanalrohrs über den Dichtabschnitt hinweg fortsetzt, wobei die Nut in der zweiten Stellung des Dichtabschnitts nicht mit dem Nebenkanalrohr oder dessen Öffnung in Fluidverbindung steht.

In einigen beispielhaften erfindungsgemäßen Ausführungsformen weist der Dichtabschnitt zusätzlich zur stirnseitigen Abdichtfläche oder zusätzlich zur Dichtnase eine erhabene, geschlossene Dichtstruktur auf, welche in der zweiten Stellung des Dichtabschnitts die Öffnung des Nebenkanalrohrs verschließt oder einen Austritt von Fluid aus der Öffnung verhindert.

In einigen beispielhaften erfindungsgemäßen Ausführungsformen ist der Dichtabschnitt als ein separates Dichtelement ausgestaltet.

In einigen beispielhaften erfindungsgemäßen Ausführungsformen weist der Dichtabschnitt wenigstens ein durchstechbares Septum auf.

In einigen beispielhaften erfindungsgemäßen Ausführungsformen sind sowohl die Septummündung als auch die Nebenkanalbohrung gemeinsam in einer Querschnittshälfte des Hauptkanals angeordnet.

In einigen beispielhaften erfindungsgemäßen Ausführungsformen weist der Steckkonnektor eine Aufnahme zum, insbesondere vorübergehenden, Aufnehmen oder Befestigen einer Schutzkappe, insbesondere vorgesehen zum Bedecken eines Nebenkanalrohrs oder eines Nebenkanalkonnektors, am Steckkonnektor auf.

Der Steckkonnektor weist vorzugsweise einen Fluidführungsabschnitt auf, durch welchen hindurch Fluid durch den Steckkonnektor in einer Längsrichtung hiervon strömen kann. Der Fluidführungsabschnitt legt damit die Längsrichtung oder Längsachse des Steckkonnektors fest.

Der Fluidführungsabschnitt weist einen ersten Endbereich und einen diesem gegenüberliegenden zweiten Endbereich auf. Sowohl der erste Endbereich als auch der zweite Endbereich können den Fluidführungsabschnitt mit dem Äußeren des Grundkörpers verbinden.

Der Fluidführungsabschnitt kann optional den geringsten Durchmesser aller im Gebrauch in der Längsrichtung des Grundkörpers von Fluid durchflossenen Strukturen des Grundkörpers aufweisen.

In exemplarischen Ausführungsformen ist der durchströmte Durchmesser oder Innendurchmesser des Fluidführungsabschnitts kleiner als der Innendurchmesser des ersten Verbindungsabschnitts.

In exemplarischen Ausführungsformen kann der durchströmte Durchmesser oder Innendurchmesser des Fluidführungsabschnitts kleiner als der Innendurchmesser der rohrförmigen Aufweitung des zweiten Verbindungsabschnitts sein.

Sowohl das erste Rastelement als auch das zweite Rastelement können eine Breite aufweisen, welche - beispielsweise als Länge oder Umfangsgrad angegeben -jeweils nur einen Bruchteil des Umfangs des Steckkonnektors ausmacht.

Zum Herstellen einer Fluidverbindung zwischen dem Fluidführungsabschnitt und dem Fluidrohr des Konnektionsabschnitts weist der Steckkonnektor in manchen erfindungsgemäßen Ausführungsformen jeweils zwischen dem äußeren Umfang der rohrartigen Aufweitung des zweiten Verbindungsabschnitts und jeweils den Rastelementen einen Freiraum auf. Dieser Freiraum ist optional und vorgesehen, um hierin einen Abschnitt eines äußeren Rohrs des Konnektionsabschnitts aufzunehmen.

Die rohrförmige Aufweitung kann ausgestaltet sein, um sowohl das Dichtelement als zumindest auch Abschnitte des Fluidrohrs des Konnektionsabschnitts in ihrem Inneren aufzunehmen.

Das Dichtelement weist in einigen erfindungsgemäßen Ausführungsformen eine Durchgangsöffnung auf, welche optional wenigstens zwei voneinander verschiedene Öffnungsquerschnitte oder Innendurchmesser aufweist. Das Dichtelement kann optional zusätzlich wenigstens zwei voneinander verschiedene Außendurchmesser aufweisen.

Zudem kann das Dichtelement an seinem im Gebrauch dem zweiten Endbereich anliegenden Ende einen Umfangswulst aufweisen. Der Umfangswulst kann, wenn er in eine wiederum optionale Umfangsrille am Innenumfang des zweiten Verbindungsabschnitts eingeführt ist, eine weitere Verbesserung der Dichtwirkung ergeben.

Der erfindungsgemäße Steckkonnektor kann genau zweiteilig sein und neben dem Grundkörper allein noch ein Dichtelement aufweisen.

Die rohrförmige Aufweitung hat in manchen erfindungsgemäßen beispielhaften Ausführungsformen mehr als nur einen Durchmesser, von denen jeder größer ist als der Innendurchmesser des zweiten Endbereichs oder des Fluidführungsabschnitts.

Einige oder alle erfindungsgemäßen Ausführungsformen können einen oder mehrere der oben oder im Folgenden genannten Vorteile aufweisen.

Der erfindungsgemäße Steckkonnektor kann Vorteile gegenüber dem Stand der Technik bieten, welche darauf beruhen, dass er kein Gewinde und/oder keine Überwurfmutter aufweist oder benötigt. Letzteres erlaubt vorteilhaft eine manuelle Ausrichtung des Steckkonnektors relativ zu einer Blutbehandlungsvorrichtung, welche den Konnektionsabschnitt aufweist. Denn anders als beispielsweise beim Festziehen der Überwurfmutter verbleibt der Steckkonnektor in der gewünschten Drehstellung oder Position. Das bei Überwurfmuttern beobachtete ungewollte Verdrehen des Konnektors, da in den letzten ca. 30 Grad des Festziehens eine konkurrierende Reibungssituation zwischen der Überwurfmutter und dem Konnektor einerseits und dem Konnektor und der Blutbehandlungsvorrichtung andererseits auftritt, tritt erfindungsgemäß nicht auf. Damit bleibt die gewünschte Sollposition oder Drehorientierung, mit welcher der Steckkonnektor auf den Konnektionsabschnitt aufgesetzt wird, vorteilhaft beibehalten. Weitere Funktionen können daher gut integriert werden, etwa ein Stechseptum im Konnektor. Denn dieses benötigt oftmals eine genaue Drehorientierung zum Dialysator und zur Blutbehandlungsapparat, nicht zuletzt wegen der beengten Raumverhältnisse.

Ferner erspart der erfindungsgemäße Steckkonnektor dem Benutzer ein mühevolles Aufschrauben, bei welchem zum Teil unerwünscht hohe Drehmomente aufgebracht werden können mit der Gefahr einer Schädigung.

Vorteilhafterweise wird passiver Berührschutz der relevanten Bereiche der Verbindungs- oder Konnektionsmündung erreicht. Daher ist keine Berührschutzkappe erforderlich. Eine Kontamination durch Fehler im üblichen Umgang oder Handling wird vorteilhaft verhindert.

Vorteilhafterweise muss der erfindungsgemäße Steckkonnektor kein Material aufweisen, welches Weichmacher aufweist. Materialien mit Weichmacherzusatz sind bei Konnektoren des Standes der Technik bekannt. Sie dienen der besseren Abdichtung beim Aufschrauben des Konnektors mit ergonomisch verträglichem Drehmoment. Sie sind mangels einer Schraubverbindung nicht erforderlich.

Vorteilhafterweise kann der erfindungsgemäße Steckkonnektor zum Verbinden von beliebig kurzen Schläuchen verwendet werden, da ein Verdrillen, was zur Schlauchknickung führen könnte, nicht auftritt.

Der erfindungsgemäße Steckkonnektor ist hinsichtlich seines Handlings optimiert. Er ist vorteilhaft leicht zu verbinden und zu lösen, was einhändig und ohne den hohen Platzbedarf für die Hand oder Hände erfolgen kann, anders als man dies beim Betätigen von bekannten Gewindeverbindern kennt.

Der erfindungsgemäße Steckkonnektor wird allein mittels Steckens und Verrastens verbunden. Der Abschluss des Verbindungsvorgangs wird dem Benutzer klar mitgeteilt, etwa durch ein hörbares und/oder am Steckkonnektor erfühlbaren Klicken, nämlich wenn die Rastelemente über diese Vorsprünge des Konnektionsabschnitts gleiten und die Vorsprünge in die Vertiefungen der Rastelemente (oder Vorsprünge der Rastelement in Vertiefungen des Konnektionsabschnitts) rasten. Hierdurch erhält der Benutzer eine zuverlässige Rückmeldung darüber, ob die gewünschte Verbindung erzielt ist oder nicht.

Die (Störfall-)Demontage des erfindungsgemäßen Steckkonnektors erfolgt aktiv durch manuelle Spreizung der Rastelemente (welche z. B. Schnappzungen oder kippbare Abschnitte als elastische, erste und zweite Abschnitte aufweisen können) oder durch ihr Zusammendrücken, je nach erfindungsgemäßer Ausgestaltung. Eine Selbstdemontage ist hingegen nicht möglich. Die Verrastung ist ferner optisch unmittelbar überprüfbar.

Der erfindungsgemäße Steckkonnektor weist in seinem Inneren keine Strömungstotzone, Toträume oder scharfe Kanten auf. Dies trägt zu guter Entlüftung, Spülung und Hämokompatibilität bei. Dies gilt auch für den Übergang zwischen Fluidführungsabschnitt und Fluidrohr des Konnektionsabschnitts.

In beispielhaften erfindungsgemäßen Ausführungsformen ist der Durchmesser des Fluidführungsabschnitts, welcher auch als Kanaldurchmesser bezeichnet werden kann, konstant; in manchen erfindungsgemäßen Ausführungsformen ist er vergleichsweise gering (z. B. zwischen 3,5 und 5,0 mm, vorzugsweise 4,0 bis 4,5 mm, besonders bevorzugt 4,2 mm, vorzugsweise jedenfalls geringer als 6,0 mm). Beides vermindert die Austrittswahrscheinlichkeit von Restflüssigkeit bei der Dekonnektion des Steckverbinders.

In beispielhaften erfindungsgemäßen Ausführungsformen ist das Dichtelement, beispielsweise der Dichtring, aus Siliconkautschuk. Diese Materialwahl trägt dazu bei, dass der Steckkonnektor gut automatisch montierbar und unproblematisch dampfsterilisierbar ist. Diese Materialwahl trägt zudem zu einer toleranzunempfindlichen Radialdichtung und aufgrund der sehr ausgeprägten axialen Nachgiebigkeit des Dichtelements zur totraumfreien Überbrückung von Maßtoleranzen bei.

Der Materialeinsatz an Siliconkautschuk beträgt dabei nur etwa ein Drittel dessen, was beispielsweise bei bekannten Rückschlagventilen erforderlich ist. Der erfindungsgemäße Steckkonnektor zeichnet sich daher durch einen günstigen Herstellpreis aus.

In beispielhaften erfindungsgemäßen Ausführungsformen ist der zweite Endbereich des Fluidführungabschnitts, welcher im Gebrauch in Fluidkommunikation mit entweder dem Dichtelement oder, falls kein Dichtelement vorgesehen ist, mit dem Stirnbereich des Fluidrohrs des Konnektionsabschnitts verbunden ist, gegenüber der rohrförmigen Aufweitung zurückversetzt. Letzteres sorgt für einen permanenten Berührschutz der bei der Konnektion relevanten Oberflächen des Steckkonnektors. Auf eine Schutzkappe für den Fluidführungsabschnitt kann vorteilhaft verzichtet werden. Diese Lösung bietet Schutz auch vor Husttropfen.

In beispielhaften erfindungsgemäßen Ausführungsformen sind der Grundkörper und, falls vorhanden, die Septumhaube aus PP (Polypropylen).

Das Septum kann aus bekanntem Septumgummi sein.

In einigen beispielhaften erfindungsgemäßen Ausführungsformen liegt das Septum vorteilhaft bündig und ohne Innendurchmessersprung und deshalb totraumfrei am Fluidführungsabschnitt, welcher beispielhaft als Blutkanal ausgestaltet ist, an.

In manchen beispielhaften erfindungsgemäßen Ausführungsformen bleibt der Zugang für den Benutzer zum Septum vorteilhaft in einer konstanten Drehstellung bezogen auf den Konnektionsabschnitt; ein Mitrotieren des Septumzugangs tritt bei Einsatz des erfindungsgemäßen Steckkonnektors anders als bei Konnektoren, die durch Vorsehen eines Gewindes zur Verbindung rotiert werden, vorteilhaft nicht auf.

In bestimmten beispielhaften erfindungsgemäßen Ausführungsformen ist der Deckel über ein Film-Schnapp-Gelenk mit dem Grundkörper des Steckkonnektors verbunden. Dies erlaubt einen ausgeprägten Schutz vor Anhusten des Septums. Zudem kann so der Zeitraum der sicheren Keimfreiheit verlängert werden. Doch auch bereits aufgrund seiner gegenüber der Oberfläche des Grundkörpers zurückversetzten Position kann das Septum vorteilhaft als permanent berührgeschützt gelten und dennoch ohne Mühe sprühdesinfiziert werden.

In einigen beispielhaften erfindungsgemäßen Ausführungsformen ist die Septumhaube einfach und kostengünstig mittels Auf-Zu-Werkzeugen gefertigt. Sie erlaubt dennoch eine unlösbare und mechanisch genaue Verrastung mit dem Steckkonnektor.

In manchen erfindungsgemäßen Ausführungsformen weist die Septumhaube wengistens ein Element auf, mittels welchem eine Verpressung des Septums erfolgt. Diese erfolgt vorzugsweise dauerhaft. Die Verpressung schützt das Septum davor, ungewollt gelöst oder undicht zu werden, insbesondere durch einen im an das Septum angrenzenden Fluidführungsabschnitt herrschenden Flüssigkeitsdruck. Gleichzeitig bedarf es keiner weiteren Befestigungslösungen für das Septum.

In beispielhaften erfindungsgemäßen Ausführungsformen ist vorteilhaft gute Dampfsterilisierbarkeit aller Räume gegeben, da in diesen Ausführungsformen an den flächigen Berührzonen der Gummiteile zu den Hartteilen Durchbrüche und/oder Drainagestrukturen vorgesehen sind.

In einigen erfindungsgemäßen Ausführungsformen ist der Steckkonnektor durch Aufstecken und Einrasten verbindbar, ohne dass es einer Drehbewegung etwa wegen einer Gewindeverbindung bedarf. Damit kann eine verbindliche Drehausrichtung vorgegeben werden; der Steckkonnektor wird nach seinem Aufsetzen nicht ungewollt rotiert beim Versuch, die Gewindeverbindung durch Drehbewegungen herzustellen.

Dabei kann ein Aufstecken unter einer unzulässigen Ausrichtung mit anschließend erforderlichem Verdrehen des Schlauchs vorteilhaft verhindert werden. Zudem tritt kein Verdrillen mit Erzeugen einer Rückstellkraft auf. Einem unbeabsichtigten Lösen der Verbindung kann somit vorgebeugt werden. Somit können auch sehr kurze Schläuche (100 mm lang oder kürzer) verwendet werden. Es ist zu ihrem Verbinden keine Drehbewegung erforderlich. Es wird damit, selbst beim Verbinden von sehr kurzen Schläuchen, kein Verdrehen (kinking) erzeugt. Kurze Schläuche ermöglichen das Einsparen von Schlauchmaterial. Ferner wird weniger Blut extrakorporal geführt als bei bekannten, längeren Schläuchen. Man kann also vorteilhaft auf lange Schläuche verzichten, die z. T. deshalb so lang waren, um ein Selbstlösen zu verhindern, was grundsätzlich einen Vorteil dargestellt hat. Dieser Vorteil wurde jedoch durch entsprechend großen Materialeinsatz und die Tatsache, dass lange Schläuche schwieriger zu handhaben sind, da sie sich beispielsweise beim Schließen von Türen, Klappen usw. der Behandlungsvorrichtung in diesen verklemmen können, etwa beim Verpressen der Blutkassette hinter einer Tür der Behandlungsvorrichtung, aufgehoben.

Die beiden letztgenannten Nachteile können erfindungsgemäß vermindert oder aufgehoben werden.

In einigen beispielhaften erfindungsgemäßen Ausführungsformen sind zusätzliche Schlauchanschlüsse für Luer-Zugabeschläuche oder mit direkten Luer-Stutzen optional einfach in den Grundkörper des Steckkonnektors integriert oder integrierbar.

In einigen beispielhaften erfindungsgemäßen Ausführungsformen sind die Rastelemente symmetrisch, bevorzugt spiegelsymmentrisch, zueinander vorgesehen. Diese Symmetrie erlaubt es vorteilhaft auf einfache Weise und ohne Verspannung, die Rastelemente zum Lösen der Verbindung in Einhandbedienung zu betätigen.

Der erfindungsgemäße Steckkonnektor erlaubt aufgrund des integrierten Septums einen hohen Nutzen im Vergleich zur aktuell sehr ungünstigen bekannten Reihenanordnung aus einer bekannten Septum-Schlauchzugabestelle und mit einem bekannten Schraubkonnektor in einem Verbindungsschlauch.

Die vorliegende Erfindung wird im Folgenden anhand der beigefügten Zeichnungen, in welcher identische Bezugszeichen gleiche oder ähnliche Bauteile bezeichnen, exemplarisch erläutert. In den zum Teil stark vereinfachten Figuren gilt:
- Fig. 1: zeigt einen Grundkörper eines erfindungsgemäßen Steckkonnektor einer ersten erfindungsgemäßen Ausführungsform in perspektivischer Ansicht;
- Fig. 2: zeigt ein Dichtelement zur Verwendung mit dem Grundkörper der Fig. 1 in Seitenansicht;
- Fig. 3: zeigt im leicht perspektivischen Längsschnitt den Steckkonnektor der ersten erfindungsgemäßen Ausführungsform mit dem Grundkörper der Fig. 1 und dem Dichtelement der Fig. 2, wobei der Steckkonnektor mit einem Konnektionsabschnitt verbunden ist;
- Fig. 4: zeigt perspektivisch einen Grundkörper eines erfindungsgemäßen Steckkonnektors einer zweiten erfindungsgemäßen Ausführungsform mit einer zusätzlichen Öffnung als weitere Fluidverbindung zu einem Fluidführungsabschnitt des Grundkörpers;
- Fig. 5: zeigt perspektivisch ein Dichtelement zur Verwendung mit dem Grundkörper der Fig. 4;
- Fig. 6: zeigt perspektivisch eine Septumhaube zur Verwendung mit dem Grundkörper der Fig. 4;
- Fig. 7: zeigt im Längsschnitt den Steckkonnektor der zweiten erfindungsgemäßen Ausführungsform mit dem Grundkörper der Fig. 4, dem Dichtelement der Fig. 5 und der Septumhaube der Fig. 6, wobei der Steckkonnektor mit einem Konnektionsabschnitt verbunden ist;
- Fig. 8: zeigt den Steckkonnektor der Fig. 7 mit Septumhaube, aber ohne Deckel, in Perspektive;
- Fig. 9: zeigt den Steckkonnektor 100 der Fig. 8 mit geschlossenem Deckel der Septumhaube, welche jener der Fig. 6 ähnlich ist;
- Fig. 10: zeigt den Steckkonnektor 100 der Fig. 9 mit Septumhaube bei geöffnetem Deckel;
- Fig. 11: zeigt den Steckkonnektor in einer dritten, erfindungsgemäßen Ausführungsform in perspektivischer Ansicht verbunden mit einer erfindungsgemäßen Blutbehandlungsvorrichtung;
- Fig. 12a, b: zeigen den Grundkörper eines Steckkonnektors in einer vierten, erfindungsgemäßen Ausführungsform als Teil eines erfindungsgemäßen Sets in Draufsicht und in zwei unterschiedlichen Stellungen;
- Fig. 13a, b: zeigen den Grundkörper eines Steckkonnektors in einer fünften, erfindungsgemäßen Ausführungsform als Teil eines erfindungsgemäßen Sets in Draufsicht und in zwei unterschiedlichen Stellungen;
- Fig. 14: zeigt das Dichtelement der Fig. 2 und seine Anordnung im Grundkörper gemäß Fig. 3; und
- Fig. 15: zeigt ein zu jenem der Fig. 2 alternatives Dichtelement und seine Anordnung in einem zu jenem der Fig. 3 alternativem Grundkörper des erfindungsgemäßen Steckkonnektors.

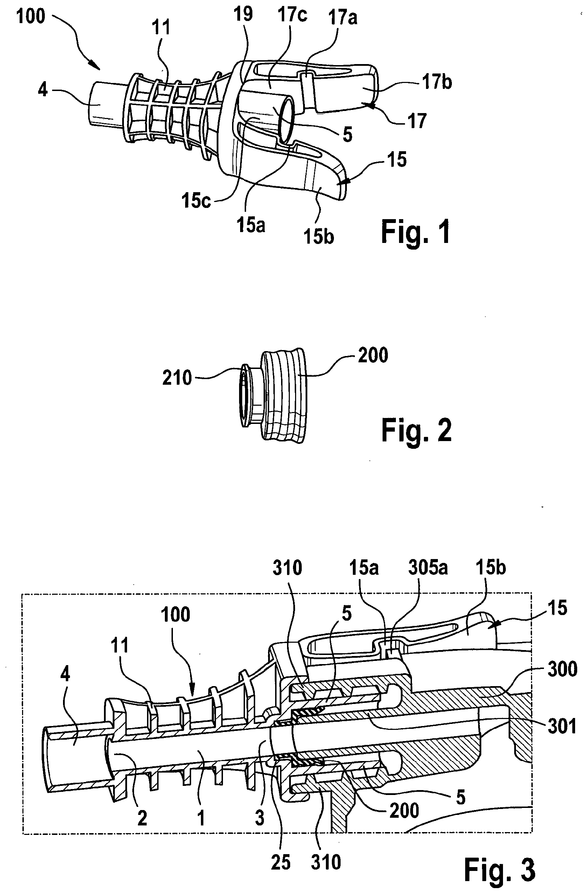

**Fig.** 1 zeigt den Grundkörper eines erfindungsgemäßen, beispielhaften Steckkonnektors 100 in einer ersten erfindungsgemäßen Ausführungsform in perspektivischer Ansicht. Fig. 1 ist im Folgenden auch unter Bezugnahme zu Fig. 2 und Fig. 3 beschrieben.

Der Steckkonnektor 100 weist einen Fluidführungsabschnitt 1 auf, durch welchen hindurch Fluid durch den Steckkonnektor 100 in einer Längsrichtung hiervon strömen kann. Der Fluidführungsabschnitt 1 legt damit die Längsrichtung oder Längsachse des Steckkonnektors 100 fest.

Der Fluidführungsabschnitt 1 weist einen ersten Endbereich 2 und einen diesem gegenüberliegenden zweiten Endbereich 3 auf. Sowohl der erste Endbereich 2 als auch der zweite Endbereich 3 können den Fluidführungsabschnitt 1 mit dem Äußeren des Grundkörpers verbinden, wie in Fig. 1 gezeigt.

Der Fluidführungsabschnitt 1 kann als Kanal ausgestaltet sein. Er kann optional, wie in Fig. 1 gezeigt, den geringsten Durchmesser aller im Gebrauch in der Längsrichtung des Grundkörpers von Fluid durchflossenen Strukturen des Grundkörpers aufweisen.

Der Steckkonnektor 100 weist einen ersten Verbindungsabschnitt 4 im Bereich des ersten Endbereichs 2 und einen zweiten Verbindungsabschnitt 5 im Bereich des zweiten Endbereichs 3 auf, welche zum Teil erstmals in Fig. 3 zu sehen sind. Der zweite Verbindungsabschnitt 5 ist exemplarisch als rohrförmige Aufweitung ausgestaltet die hier ebenfalls mit dem Bezugszeichen 5 adressiert wird.

Der erste Verbindungsabschnitt 4 dient zur fluidischen Verbindung des Steckkonnektors 100, seines ersten Endbereichs 2 oder des Fluidführungsabschnitts 1 mit einem Fluidsystem, etwa mit dem Ende einer Schlauchleitung.

Der zweite Verbindungsabschnitt 5 dient zur fluidischen Verbindung des Steckkonnektors 100, seines zweiten Endbereichs 3 oder des Fluidführungsabschnitts 1 mit einem Fluidrohr 301 des erstmals in Fig. 3 gezeigten Konnektionsabschnitts 300. In der exemplarischen Ausführungsform der Fig. 1 ist die fluidische Verbindung zwischen dem zweiten Endbereich 3 und dem Konnektionsabschnitt 300 eine mittelbare, nicht aber eine unmittelbare, da der zweite Endbereich 3 in unmittelbarem Kontakt mit einem in Fig. 2 erstmals gezeigten Dichtelement 200 steht, nicht aber unmittelbar mit dem Konnektionsabschnitt 300 selbst. Es ist das Dichtelement 200, welche seinerseits unmittelbar mit dem Konnektionsabschnitt 300 Kontakt hat und mit diesem in Fluidverbindung steht.

Der zweite Verbindungsabschnitt 5 dient als Aufnahmeabschnitt zum Aufnehmen eines Endes des Fluidrohrs 301.

In der beispielhaften Ausführungsform der Fig. 1 schließt sich der zweite Verbindungsabschnitt 5, welcher hier beispielhaft als rohrförmige Aufweitung 5 ausgestaltet ist an den zweiten Endbereich 3 unter größerem Abstand zum ersten Endbereich 2 als zum zweiten Endbereich 3 an.

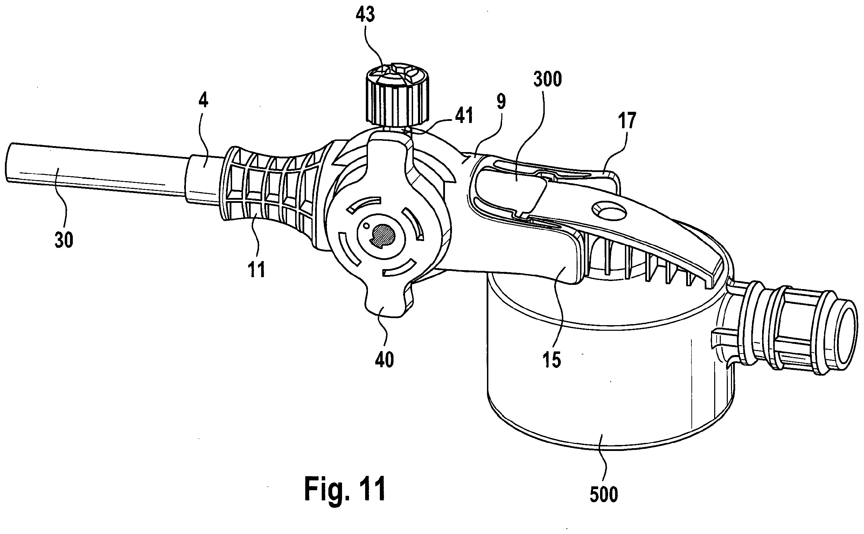

In exemplarischen Ausführungsformen wie der in Fig. 1 gezeigten ist der durchströmte Durchmesser oder Innendurchmesser des Fluidführungsabschnitts 1 kleiner als der Innendurchmesser des ersten Verbindungsabschnitts 4. So kann beispielsweise ein Ende eines erstmals in Fig. 11 gezeigten Schlauchs 30 des nicht gezeigten Fluidsystems unter Verkleben oder Verklemmen in den ersten Verbindungsabschnitt 4 und damit in den Grundkörper eingeführt werden. Er kann aufgrund dieser Ausgestaltung derart mit letzterem verbunden werden, dass es nicht zu einem Durchmessersprung im Übergang von Schlauchende und Fluidführungsabschnitt 1 kommen muss. Letzteres dient vorteilhaft der Verhinderung von Turbulenzen aufgrund sich sprunghaft ändernder Strömungsquerschnitte und der Verhinderung von Toträumen.

In exemplarischen Ausführungsformen wie der in Fig. 1 gezeigten ist ergänzend oder alternativ zu dem im vorangegangenen Absatz Gesagten der durchströmte Durchmesser oder Innendurchmesser des Fluidführungsabschnitts 1 kleiner als der Innendurchmesser der rohrförmigen Aufweitung des zweiten Verbindungsabschnitts 5. Ist, wie beim exemplarischen Ausführungsbeispiel der Fig. 1 zu sehen, der Innendurchmesser der rohrförmigen Aufweitung größer als jener des Fluidführungsabschnitts 1, so kann beispielsweise ein Dichtelement 200, welches erstmals in Fig. 2 gezeigt ist, wiederum in die rohrförmige Aufweitung und damit in den Grundkörper eingeführt und beispielsweise durch Verklemmen oder Verrasten mit diesem verbunden werden, ohne dass es zu einem Durchmessersprung im Übergang von Dichtelement 200 und Fluidführungsabschnitt 1 kommen muss. Letzteres dient wiederum vorteilhaft der Verhinderung von Turbulenzen aufgrund sich sprunghaft ändernder Strömungsquerschnitte und der Verhinderung von Toträumen. Ferner erlaubt diese Ausgestaltung vorteilhaft, das Dichtelement 200, wie Fig. 3 zu entnehmen sein wird, ohne Veränderung des durchströmten Durchmessers im Bereich des Dichtelements 200 in wenigstens zwei unterschiedlichen Bereichen hiervon in Kontakt mit dem erstmals in Fig. 3 gezeigten Konnektionsabschnitt 300 zu bringen. Letzteres trägt vorteilhaft zu erhöhter Dichtwirkung bei.

Der Steckkonnektor 100 weist ein erstes Rastelement 15 und ein rein optional vorgesehenes zweites Rastelement 17 auf. In der exemplarischen Ausführungsform der

Fig. 1 ragen beide in Längsrichtung des Grundkörpers über ein freies Ende des zweiten Verbindungsabschnitts 5 vor.

Das erste Rastelement 15 ist in einem ersten Abschnitt 15b hiervon, also dem in Fig. 1 rechts gezeigten Ende des Rastelements 15, elastisch ausgestaltet. Optional ist auch das zweite Rastelement 17 in einem zweiten Abschnitt 17b hiervon, dem in Fig. 1 rechts gezeigten Ende des Rastelements 17, elastisch ausgestaltet. Der erste und der zweite Abschnitt 15b, 17b können aufgrund ihrer Elastizität unter Kraftaufbringung in gewissem Maße voneinander wegbewegt werden, um sich nach Aufheben der Kraft wieder aufeinander zu zu bewegen. Optional handelt es sich beim ersten Abschnitt 15b und beim zweiten Abschnitt 17b um jene Abschnitte des ersten bzw. zweiten Rastelements 15, 17, mittels welcher die letztgenannten über das freie Ende des zweiten Verbindungsabschnitts 5 überstehen.

Sowohl das erste Rastelement 15 als auch das zweite Rastelement 17 weisen eine Breite auf, welche - beispielsweise als Länge oder Umfangsgrad angegeben - jeweils nur einem Bruchteil des Umfangs des Steckkonnektors 100 in einem dritten Bereich 19 ausmacht, in welchem sowohl das erste Rastelement 15 als auch das optionale zweite Rastelement 17 mit den übrigen Steckkonnektorabschnitten verbunden sind. Anders angedrückt stehen die beiden Rastelemente 15, 17 "auf Lücke"; zwischen ihnen ist in Umfangsrichtung des Steckkonnektors 100 Platz.

Sowohl das erste Rastelement 15 als auch das zweite Rastelement 17 weisen in der exemplarischen Ausführungsform der Fig. 1 je eine Vertiefung 15a bzw. 17a auf. Beide Vertiefungen 15a, 17a sind vorgesehen, um einen oder mehrere Vorsprünge 305a des Konnektionsabschnitts 300, von welchen einer erstmals in Fig. 3 gezeigt ist, aufzunehmen. Durch Aufnehmen der Vorsprünge 305a in den Vertiefungen 15a und 17a - und ggf. durch Führen eines Fluidrohrs 301 des Konnektionsabschnitts 300 im Inneren des hier exemplarisch als rohrförmige Aufweitung 5 ausgestalteten zweiten Verbindungsabschnitts 5 als Aufnahmeabschnitt sowie durch Aufschieben eines erstmals in Fig. 3 gezeigten äußeren Rohrs 310 über die rohrförmige Aufweitung 5 wird der Konnektionsabschnitt 300 am Steckkonnektor 100, und umgekehrt, festgelegt.

Die Verbindung zwischen den Vorsprüngen 305a und den Vertiefungen 15a und 17a ist zerstörungsfrei lösbar. Der Steckkonnektor 100 ist daher wieder verwendbar.

Zum Verbinden des Steckkonnektors 100 mit dem in Fig. 3 gezeigten Konnektionsabschnitt 300 wird der Steckkonnektor 100 auf den Konnektionsabschnitt 300 aufgeschoben. Dabei vergrößert sich der Abstand zwischen dem ersten Rastelement 15 und dem zweiten Rastelement 17 aufgrund der spreizenden Wirkung der Vorsprünge 305a solange weiter zunehmend, bis die Vertiefungen 15a und 17a über die Vorsprünge 305a gleiten und diese aufgrund der Elastizität in sich aufnehmen. Ist dies geschehen, so ist der Vorgang des Verbindens, was erkennbar ein Verrasten ist oder umfasst, beendet. Die gewünschte Verbindung ist hergestellt.

Zum Lösen der Verbindung werden das erste Rastelement 15 und, sofern vorhanden, das zweite Rastelement 17 mit der Hand gespreizt, sprich gegen den von ihnen ausgehenden elastischen Widerstand verformt, so dass sich der Abstand zwischen dem ersten Abschnitt 15b und dem zweiten Abstand 17b so weit vergrößert, bis ihre Vertiefung 15a bzw. 17a die Vorsprünge 305a wieder ausreichend freigeben, um den Konnektionsabschnitt 300 durch Ziehen vom Steckkonnektor 100, oder umgekehrt, lösen zu können.

Der Fluidführungsabschnitt 1 kann an seinem Umfang von einer Steg-Waben-Struktur 11 umgeben sein oder eine solche aufweisen. Sie trägt aufgrund ihrer unhomogenen Oberflächentextur dazu bei, dass man den Steckkonnektor 100 sicher mit der Hand fassen kann. Zugleich stützt sie den Fluidführungsabschnitt 1 und schützt ihn von mechanischer Beschädigung, etwa durch Abknicken.

Zum Herstellen einer Fluidverbindung zwischen dem Fluidführungsabschnitt 1 und dem Fluidrohr 301 des Konnektionsabschnitts 300 weist der Steckkonnektor 100, wie Fig. 1 zu entnehmen ist, jeweils zwischen dem äußeren Umfang der rohrartigen Aufweitung des zweiten Verbindungsabschnitts 5 und jeweils den Rastelementen 15, 17 einen Freiraum 15c, 17c auf. Dieser Freiraum 15c, 17c ist optional und vorgesehen, um hierin Abschnitte des in Fig. 3 erstmals gezeigten äußeren Rohrs 310 des Konnektionsabschnitts 300 aufzunehmen. Das äußere Rohr 310 umgibt das in seinem Inneren liegende Fluidrohr 301 des Konnektionsabschnitts 300, beispielsweise auf konzentrische Weise.

In manchen erfindungsgemäßen Ausführungsformen besteht der erfindungsgemäße Steckkonnektor 100 aus dem Grundkörper, wie er in Fig. 1 gezeigt ist, oder in anderer Ausgestaltung. In anderen Ausführungsformen weist der Steckkonnektor 100 neben dem Grundkörper zumindest ein Dichtelement auf, wie es in einer beispielhaften Ausgestaltung in Fig. 2 gezeigt ist. Zu den letztgenannten, beispielhaften Ausgestaltungen zählt jene der Fig. 1, in welcher ein Dichtelement 200 vorgesehen ist, beispielsweise ausgestaltet wie in Fig. 2 zu erkennen.

Die rohrförmige Aufweitung 5 ist ausgestaltet, um sowohl das Dichtelement 200 als zumindest auch Abschnitte des Fluidrohrs 301 des Konnektionsabschnitts 300 in ihrem Inneren aufzunehmen, wie dies in Fig. 3 zu erkennen ist.

**Fig.** 2 zeigt ein Dichtelement 200, welches optional als Dichtring ausgestaltet ist, und welches vorgesehen ist, um in den in die rohrförmige Aufweitung des zweiten Verbindungsabschnitts 5 des Grundkörpers des Steckkonnektors 100, siehe Fig. 1 und Fig. 3, eingesteckt zu werden.

Das Dichtelement 200 weist im Beispiel der Fig. 1 eine Durchgangsöffnung auf, welche optional wenigstens zwei voneinander verschiedene Öffnungsquerschnitte oder Innendurchmesser aufweist. Das Dichtelement 200 kann optional zusätzlich wenigstens zwei voneinander verschiedene Außendurchmesser aufweisen. Beides ist in Fig. 2 zu erkennen.

Zudem kann das Dichtelement 200 an seinem im Gebrauch dem zweiten Endbereich 3 anliegenden Ende einen Umfangswulst 210 aufweisen. Der Umfangswulst 210 kann, wenn er in eine wiederum optionale Umfangsrille 25 am Innenumfang des zweiten Verbindungsabschnitts 5 eingeführt ist, eine weitere Verbesserung der Dichtwirkung ergeben.

**Fig.** 3 zeigt den Steckkonnektor 100 der Fig. 1 mit dem Dichtelement 200 aus Fig. 2, wobei der Steckkonnektor 100 mit dem oben bereits genannten Konnektionsabschnitt 300 in bestimmungsgemäßer Fluidverbindung verbunden ist. Fig. 3 zeigt damit das erfindungsgemäße Set in einer beispielhaften Ausführungsform.

In Fig. 3 ist zu erkennen, wie die Vertiefung 15a des ersten Rastelements 15 den Vorsprung 305a des Konnektionsabschnitts 300 in sich aufnimmt. Das Rastelement 15 ist mit Blick auf dessen Innenseite dargestellt. Diese kann dessen Unterseite sein.

Wie anhand Fig. 3, welche nur genau ein Rastelement zeigt, erkennbar ist, kann es in manchen erfindungsgemäßen Ausführungsformen genügen, die durch Einstecken des Fluidrohrs 301 in den zweiten Verbindungsabschnitt 5 und durch Aufstecken des äußeren Rohrs 310 auf den Außenumfang des zweiten Verbindungsabschnitt 5 erzeugte Verbindung zwischen Steckkonnektor 100 und Konnektionsabschnitt 300 mit nur genau einem Rastelement durch Verrasten zu sichern.

Das Dichtelement 200 weist erkennbar einen ersten Innendurchmesser auf (welcher am linken Ende des Dichtelements 200, bezogen auf die Fig. 3, zu sehen ist), welcher dem Innendurchmesser des zweiten Endbereichs 3 oder dem Fluidführungsabschnitt 1 des Steckkonnektors 100 entspricht. Diese Entsprechung ist nicht zwingend erforderlich, bietet jedoch den Vorteil, dass der Übergang zwischen dem zweiten Endbereich 3 und dem Dichtelement 200 strömungstechnisch vorteilhaft ist, indem beispielsweise das Auftreten von Turbulenzen des Fluids beim Übergang zwischen Dichtelement 200 und zweitem Endbereich 3 verhindert oder vermindert werden.

Der erfindungsgemäße Steckkonnektor 100 der ersten Ausführungsform kann, wie Fig. 3 zu entnehmen ist, als zweiteiliger Steckkonnektor 100 verstanden werden, welcher, falls gewünscht, ausschließlich aus seinen Grundkörper mit dem ersten und optional dem zweiten Rastelement 15, 17, wie in Fig. 1 gezeigt, sowie dem Dichtelement 200 der Fig. 2 bestehen kann.

Wie Fig. 3, aber auch bereits Fig. 1, weiter zu entnehmen ist, kann sich an den zweiten Endbereich 3 des Grundkörpers die rohrförmige Aufweitung des zweiten Verbindungsabschnitts 5 anschließen. Sie kann, wie in Fig. 3 gezeigt, der Aufnahme des Dichtelements 200 in ihrem Inneren dienen und hierzu radial, verglichen mit dem Innendurchmesser des zweiten Endbereichs 3, weiter sein, also einen größeren Durchmesser haben. Der größere Durchmesser erlaubt es, das Dichtelement 200 derart in der rohrförmigen Aufweitung und zugleich in Kontakt mit dem zweiten Endbereich 3 anzuordnen, dass ein Übergang vom zweiten Endbereich 3 zum Dichtelement 200 ohne Stufe oder Durchmesserveränderung vorgesehen sein kann.

Die rohrförmige Aufweitung 5 hat in dem in Fig. 1 und Fig. 3 gezeigten Beispiel mehr als nur einen Durchmesser, von denen jeder größer ist als der Innendurchmesser des zweiten Endbereichs 3 oder des Fluidführungsabschnitts 1. Konkret sind es beim Beispiel der Fig. 1 und der Fig. 3 wenigstens zwei solche Durchmesser, an welchen das Dichtelement 200 an je einem Umfangsabschnitt der rohrförmigen Aufweitung 5 anliegt.

Zu erkennen ist, dass das Dichtelement 200 ausgestaltet ist, um sowohl an der Stirnseite des Mündungsabschnitts des Fluidrohrs 301 als auch an einer Umfangsseite (entlang des geschlossenen Umfangs) des Fluidrohrs 301 am Fluidrohr 301 anzuliegen. Auf diese Weise besteht doppelte Dichtung, einmal an der Stirnseite, einmal am Umfang, jeweils des Fluidrohrs 301, also sowohl axial als auch radial. Möglich wird diese doppelte Dichtung durch den gestuften Aufbau des Dichtelements 200, welcher sich durch das Vorsehen von mehr als nur einem Innendurchmesser des Dichtelements 200 ergibt.

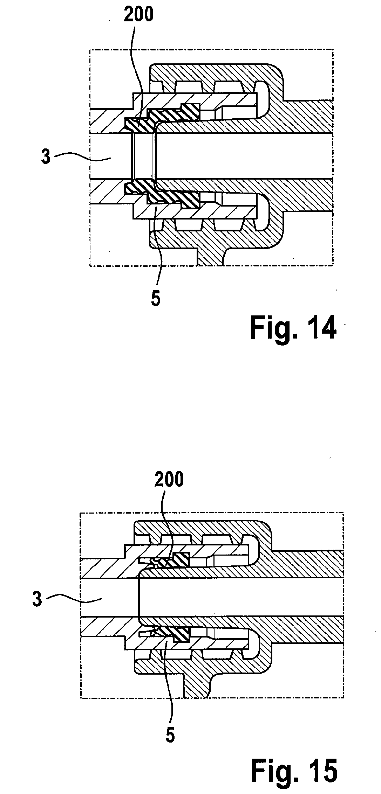

Es sei angemerkt, dass die besondere Ausgestaltung der Grundkörpers und/oder der rohrförmigen Aufweitung 5 des Grundkörpers auf die besondere Gestalt des Dichtelements 200, wie hierin beschrieben, angepasst ist. Sollte hingegen ein Dichtelement vorgesehen werden, welches im Gebrauch einen Kontakt zwischen zweitem Endbereich 3 und Stirnseite des Fluidrohrs 301 des Konnektionsabschnitts 300 nicht verhindert sondern zulässt, was in Fig. 15 gezeigt ist, so ist dies ebenfalls von der vorliegenden Erfindung umfasst.

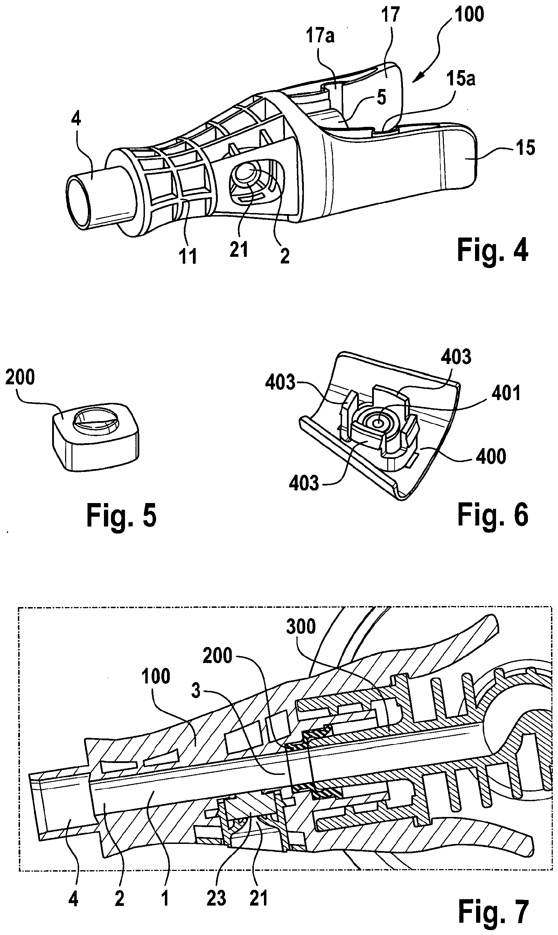

**Fig. 4** zeigt einen Grundkörper eines erfindungsgemäßen, beispielhaften Steckkonnektors 100 in einer zweiten erfindungsgemäßen Ausführungsform in perspektivischer Ansicht.

Der Steckkonnektor 100 dieser Ausführungsform weist den in Fig. 4 gezeigten Grundkörper auf. Dieser unterscheidet sich vom Grundkörper des Steckkonnektors 100 der Fig. 1 durch eine Zugabe- oder Entnahmeöffnung 21, kurz: Öffnung 21. Die Öffnung 21 stellt eine Fluidverbindung zwischen einem Äußeren des Steckkonnektors 100 und dem Inneren des Fluidführungsabschnitts 1 des Grundkörpers dar. Die Öffnung 21 dient der Zugabe beispielsweise eines Medikaments zu dem durch den Steckkonnektor 100 im Fluidführungsabschnitt 1 zwischen erstem Endbereich 2 und zweitem Endbereich 3 fließenden Fluids.

Die Öffnung 21 zum Fluidführungsabschnitt 1 kann, wie in Fig. 4 gezeigt, von einer Seite des Grundkörpers, beispielsweise einer Seite eines Griffabschnitts, zugänglich ausgestaltet sein. Die Öffnung 21 dient der Zugabe beispielsweise eines Medikaments zu dem durch den Steckkonnektor 100 im Fluidführungsabschnitt 1 zwischen erstem Endbereich 2 und zweitem Endbereich 3 fließenden Fluids.

Die Öffnung 21 liegt erkennbar in einer Vertiefung des Grundkörpers. Die Öffnung 21 wird im Gebrauch mittels eines Septums 23 verschlossen, welches erstmals in Fig. 7 zu sehen ist und beispielsweise aus einem Septumgummi bestehen kann. Die Vertiefung erzeugt einen Abstand zwischen der Öffnung 21 oder einer äußeren Oberfläche des Septumgummis oder des Septums 23 einerseits, und einer äußeren Oberfläche des Grundkörpers, beispielsweise der Steg-Waben-Struktur 11, andererseits. Mittels dieses Abstandes ist das Septum 23 permanent berührgeschützt, auch, weil die Öffnung 21 zu klein ist, um mit dem Finger in sie hineinzugreifen. Das Septum 23 ist zugleich aber zugänglich genug, um mittels Kanüle oder Sprühdesinfektions erreichbar zu sein.

**Fig. 5** zeigt ein Dichtelement 200 für den Steckkonnektor 100 der Fig. 4. Es kann optional als das Septum 23 verwendet werden.

**Fig. 6** zeigt eine Septumhaube 400, welche optional zusammen mit dem Steckkonnektor 100 der Fig. 4 verwendet werden kann. Die Septumhaube 400 kann einen Deckel 401 aufweisen. Dessen Rückseite ist in Fig. 6 im Zentrum der Septumhaube 400 zu erkennen. Der Deckel 401 ist in Fig. 6 geschlossen.

Die Septumhaube 400 ist ausgestaltet, um die Vertiefung, welche aus Sicht des Anwenders vor der Öffnung 21 oder vor dem Septum 23 liegt, gegenüber einem Äußeren des Grundkörpers des Steckkonnektors 100 abzudecken.

Das Septum 23 kann um 1 bis 15 mm zurückversetzt werden, bevorzugt um etwa 2 bis 10 mm, besonders bevorzugt um 3 bis 5 mm.

Die Septumhaube 400 kann ausgestaltet sein, um - bezogen auf eine Oberfläche des Grundkörpers, mit welcher die Septumhaube 400 bündig abschließt - einen stufenfreien Verschluss der Vertiefung zu bilden.

Zu seiner Befestigung am Grundkörper kann die Septumhaube 400 Haken, Rastnasen 403 oder Vorsprünge aufweisen. Der Grundkörper kann entsprechende Vertiefungen oder Hinterschneidungen zu deren Aufnahme oder Einrasten aufweisen. Andere Befestigungsweisen sind ebenfalls von der vorliegenden Erfindung umfasst.

**Fig. 7** zeigt in einem Längsschnitt den Steckkonnektor 100 der Fig. 4 mit einem Dichtelement 200 der Fig. 5. Beide sind bestimmungsgemäß mit dem Konnektionsabschnitt 300 verbunden. Fig. 7 zeigt damit das erfindungsgemäße Set in einer weiteren, beispielhaften Ausführungsform.

Die doppelte, d.h. sowohl radiale als auch axiale, fluidische Abdichtung des Fluidführungsabschnitts 1 gegenüber dem Fluidrohr 301 des Konnektorabschnitts 300 mittels des Dichtelements 200 ist auch der zweiten erfindungsgemäßen Ausführungsform der Fig. 7 zu entnehmen. Gleichwohl ist dieses Merkmal in jeder erfindungsgemäßen Ausführungsform optional.

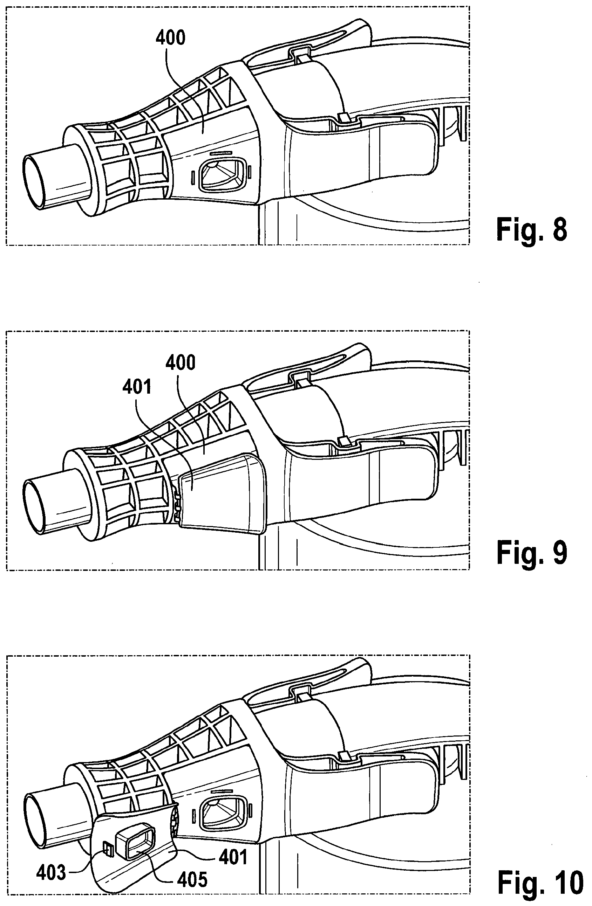

**Fig. 8** zeigt den Steckkonnektor 100 der Fig. 4 mit Septumhaube 400, jedoch ohne Deckel 401.

**Fig. 9** zeigt den Steckkonnektor 100 der Fig. 8 mit geschlossenem Deckel 401 der Septumhaube 400, welche wiederum jener der Fig. 6 ähnlich ist.

**Fig. 10** zeigt den Steckkonnektor 100 der Fig. 9 mit Septumhaube 400 bei geöffnetem Deckel 401. Zu erkennen sind eine Rastnase 403 sowie ein vom Deckel 401 vorstehender Dichtrand 405 mit umlaufendem Rand. Letzterer dient dazu, den Deckel 401 geometrisch an der Septumhaube 400 festzulegen. Ferner kann er möglicherweise helfen, die Vertiefung gegen Eintritt von Flüssigkeit abzudichten.

**Fig. 11** zeigt den Steckkonnektor 100 in einer dritten, erfindungsgemäßen Ausführungsform in perspektivischer Ansicht.

Er hat ergänzend zu den Merkmalen, welche bereits der Steckkonnektor 100 der oben diskutierten ersten oder zweiten Ausführungsform aufweist, als weitere Merkmale ein Betätigungselement 40 zum Betätigen eines im Steckkonnektor 100 vorgesehenen Mehrwegeventils. Das Mehrwegeventil dient dem Schalten zumindest eines Nebenkanals, welcher in einem Nebelkanalrohr 41 gefügt ist. Eine optionale Schutzkappe trägt das Bezugszeichen 43.

Ferner ist der Steckkonnektor 100 mittels des ersten Verbindungsbereichs 4 mit einem Schlauch 30 bzw. dem Ende einer Schlauchleitung verbunden. Ebenso ist der Konnektionsabschnitt 300 in Fluidverbindung mit einer Blutbehandlungsvorrichtung 500 verbunden. Anstelle mit der in Fig. 11 gezeigten Blutbehandlungsvorrichtung 500 verbunden zu sein, könnte der Konnektionsabschnitt 300 in Fluidverbindung mit einem Adapter verbunden oder Teil hiervon sein.

Fig. 11 zeigt den Steckkonnektor 100 mit einem mittels Betätigungselement 40 betätigbaren Ventilelement, welches in der DE 10 2011 108 787 A1 als Port beschrieben ist. Die diesbezügliche Offenbarung wird hiermit mittels Bezugsnahme vollumfänglich aufgenommen.

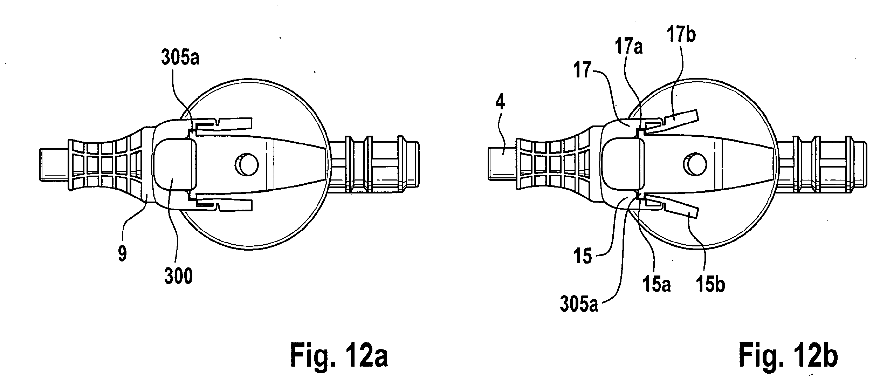

**Fig. 12a, 12b** zeigen den Grundkörper eines Steckkonnektors 100 in einer vierten, erfindungsgemäßen Ausführungsform in Draufsicht und in zwei unterschiedlichen Stellungen, von denen eine in Fig. 12a und eine in Fig. 12b gezeigt ist.

In der in Fig. 12a gezeigten Stellung ist der Verbindungsvorgang, bei welchem der Steckkonnektor 100 von links nach rechts auf den Konnektionsabschnitt 300 aufgeschoben wird, noch nicht abgeschlossen, anders als in Fig. 12b. Die in Fig. 12a gezeigte Stellung zeigt den Steckkonnektor 100 dieser exemplarischen Ausführungsform somit vor Abschluss des Verbindungsvorgangs, was zugleich aber auch dem Beginn des Lösevorgangs entspricht, also jenem Vorgang, bei welchem der Steckkonnektor 100 vom Konnektionsabschnitt 300 gelöst werden soll.

Der in Fig. 12a, b gezeigte Grundkörper unterscheidet sich hinsichtlich seines Rastelements 15, 17 vom Grundkörper jeweils der vorangegangenen Ausführungsformen dadurch, dass bei ihnen der erste elastische Abschnitt 15b und der zweite elastische Abschnitt 17b jeweils als kippbarer Abschnitt mit jeweils einem vorderen Ende und einem hinteren Ende ausgestaltet sind oder einen solchen kippbaren Abschnitt aufweisen. Das vordere Ende ist dabei dem ersten Verbindungsabschnitt 4 näher als das hintere. Das hintere Ende kann, wie beispielhaft in Fig. 12a, b gezeigt ist, den Grundkörper zu einer Seite hiervon abschließen. Der kippbare Abschnitt 15b, 17b ist jeweils relativ zum übrigen Rastelement 15, 17 oder Grundkörper gegen Materialrückstellkräfte oder Materialwiderstände kippbar oder klappbar angeordnet.

Wie im Vergleich der Fig. 12a mit der Fig. 12b zu erkennen ist, unterscheidet sich der Grundkörper in der hier gezeigten vierten Ausführungsform von jenem der vorangegangenen Figuren dadurch, dass sich die Vertiefungen 15a, 17a der Rastelemente 15, 17 erst dann einstellen, wenn keine äußeren Kräfte auf den kippbaren Abschnitt 15b, 17b als ersten bzw. zweiten Abschnitt einwirken. Zu solchen äußeren Kräften sind vorliegend Kräfte zu zählen, mit welchen der Benutzer die hinteren Enden der beiden kippbaren Abschnitte 15b, 17b aufeinander zu bewegt, und Kräfte, mit welchen die Vorsprünge 305a die vorderen Enden der beiden kippbaren Abschnitte 15b, 17b auseinander drücken.

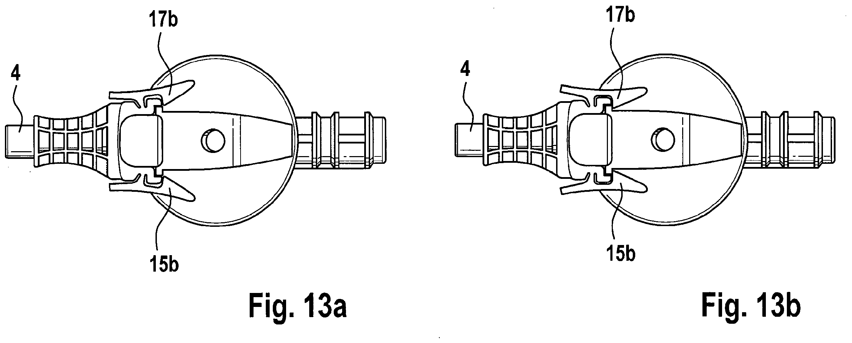

**Fig. 13a, Fig. 13b** zeigen den Grundkörper eines Steckkonnektors 100 in einer fünften, erfindungsgemäßen Ausführungsform in Draufsicht und in zwei unterschiedlichen Stellungen, von denen eine in Fig. 13a und eine in Fig. 13b gezeigt ist.

In der in Fig. 13a gezeigten Stellung ist der Verbindungsvorgang, bei welchem der Steckkonnektor 100 von links nach rechts auf den Konnektionsabschnitt 300 aufgeschoben wird, noch nicht abgeschlossen, anders als in Fig. 13b. Die in Fig. 13a gezeigte Stellung zeigt den Steckkonnektor 100 dieser exemplarischen Ausführungsform somit vor Abschluss des Verbindungsvorgangs, was zugleich aber auch dem Beginn des Lösevorgangs entspricht, also jenem Vorgang, bei welchem der Steckkonnektor 100 vom Konnektionsabschnitt 300 gelöst werden soll.

Der in Fig. 13a, b gezeigte Grundkörper unterscheidet sich hinsichtlich seines Rastelements 15, 17 vom Grundkörper der Fig. 12a, b dadurch, dass die kippbaren Abschnitte 15b, 17b durch Kraftaufbringung durch den Benutzer auf das vordere Ende geöffnet werden können, so dass der Vorsprung 305a frei kommt, wobei der Vorsprung 305a beim Verbindungsvorgang die hinteren Enden auseinander drückt.

Anders als in den Ausführungsformen der Fig. 1 bis 10 wird somit in den Ausgestaltungen der Fig. 12a, b und der Fig. 13a, b nicht sowohl beim Verbinden als auch zum Lösen der Verbindung zwischen Vertiefung 15a, 17a und Vorsprung 305a Druck auf ein und denselben Abschnitt des Rastelements 15, 17 ausgeübt. Vielmehr wird bei Vorsehen eines kippbaren Abschnitts 15b, 17b zum Verbinden Kraft auf ein erstes Ende und beim Lösen der Verbindung Kraft auf ein gegenüberliegendes Ende des kippbaren Abschnitts 15b, 17b aufgebracht. Dies erlaubt es vorteilhaft, einen Grundkörper anzubieten, bei dem je nach Vorliebe des Benutzers und unter Berücksichtung von ergonomischen und den Einbau betreffenden Umständen zum Lösen der Verbindung ein Spreizen bzw. ein Voneinanderweg-Bewegen oder ein Zusammendrücken bzw. ein Aufeinanderzu-Bewegen gefordert werden kann.

Der erste und der zweite Abschnitt 15b, 17b der Rastelemente 15, 17 sind in den Ausführungsformen der Fig. 12a, b und der Fig. 13a, b =als kippbare Abschnitte ausgestaltet.

**Fig. 14** zeigt das Dichtelement 200 der Fig. 2 und seine Anordnung im Grundkörper gemäß Fig. 3, bei welcher es einen direkten Kontakt zwischen dem Endbereich 3 und der Mündung des Fluidrohrs 301 verhindert.

**Fig. 15** zeigt ein alternatives Dichtelement 200 und seine Anordnung in einem alternativen Grundkörper des erfindungsgemäßen Steckkonnektors 100, bei welcher es einen direkten Kontakt zwischen dem Endbereich 3 und der Mündung des Fluidrohrs 301 nicht verhindert. Es ist vielmehr angeordnet, um an einem Außenumfang des Fluidrohrs 301 und an einem Innenumfang der rohrförmigen Aufweitung 5 anzuliegen, nicht aber an einer Stirnfläche des Fluidrohrs 301.

### Bezugszeichenliste

- 100: Steckkonnektor
- 1: Fluidführungsabschnitt
- 2: erster Endbereich
- 3: zweiter Endbereich
- 4: erster Verbindungsabschnitt
- 5: zweiter Verbindungsabschnitt; rohrförmige Aufweitung
- 11: Steg-Waben-Struktur
- 15: erstes Rastelement
- 15a: Vertiefung
- 15b: erster Abschnitt, kippbarer Abschnitt
- 15c: Freiraum
- 17: zweites Rastelement
- 17a: Vertiefung
- 17b: zweiter Abschnitt, kippbarer Abschnitt
- 17c: Freiraum
- 19: dritter Bereich
- 21: Zugabe- oder Entnahmeöffnung, kurz: Öffnung
- 23: Septum
- 25: Umfangsrille
- 30: Schlauch oder Schlauchleitung
- 40: Betätigungselement
- 41: Nebenkanalrohr
- 43: Schutzkappe
- 200: Dichtelement
- 210: Umfangswulst
- 300: Konnektionsabschnitt
- 305a: Vorsprünge
- 301: Fluidrohr
- 310: Äußeres Rohr
- 400: Septumhaube
- 401: Deckel
- 403: Rastnasen
- 405: Dichtrand
- 500: Blutbehandlungsvorrichtung

## Patentansprüche

1. Medizinischer Steckkonnektor (100) zum Aufstecken auf einen Konnektionsabschnitt (300) beispielsweise einer Blutbehandlungsvorrichtung (500) oder zum Aufstecken auf einen Konnektionsabschnitt (300) eines Verbindungsdapters,
wobei der Steckkonnektor (100) wenigstens einen Grundkörper mit einem Aufnahmeabschnitt zum Aufnehmen eines Endes eines Fluidrohrs (301) des Konnektionsabschnitts (300) aufweist, wobei der Grundkörper aufweist:
- ein erstes Rastelement (15) mit einem ersten Abschnitt (15b), wobei der erste Abschnitt (15b) elastisch biegbar oder elastisch kippbar ausgestaltet ist;
- optional ein zweites Rastelement (17) mit einem zweiten Abschnitt (17b);
- wobei wenigstens eines der Elemente einer Gruppe, welche aus dem ersten Rastelement (15) und dem zweiten Rastelement (17) besteht, eine Vertiefung (15a) zur Aufnahme eines Vorsprungs (305a) des Konnektionsabschnitts (300) und/oder eine Erhebung zum Eingreifen in eine Vertiefung beispielsweise der Blutbehandlungsvorrichtung (500) oder des Verbindungsadapters aufweist, wobei die Vertiefung (15a, 17a) oder die Erhebung eines Rastelements (15, 17) aus der Gruppe dem jeweils anderen Rastelement (17, 15) aus der Gruppe und/oder dem Aufnahmeabschnitt zugewandt ist, oder an einer Innenseite des jeweiligen Rastelements (15, 17) angeordnet ist,
wobei der Steckkonnektor einen Fluidführungsabschnitt (1) in seinem Grundkörper aufweist, durch welchen hindurch Fluid durch den Steckkonnektor (100) in einer Längsrichtung hiervon strömen kann,
wobei der Grundkörper aus einem ersten Material besteht oder ein solches aufweist, wobei der Steckkonnektor (100) wenigstens ein Dichtelement (200) aus einem zweiten, vom ersten Material verschiedenen Material aufweist,
dadurch charakterisiert, dass
das Dichtelement (200) eine Durchgangsöffnung aufweist mit wenigstens einem Innendurchmesser, welcher einem Innendurchmesser des Fluidführungsabschnitts (1) des Grundkörpers entspricht.

2. Steckkonnektor (100) nach einem der vorangegangenen Ansprüche, wobei der zweite Abschnitt (17b) des zweiten Rastelements (17) ebenfalls elastisch biegbar oder elastisch kippbar ausgestaltet ist.

3. Steckkonnektor (100) nach einem der vorangegangenen Ansprüche, wobei der erste Abschnitt (15b) des ersten Rastelements (15) einerseits und der zweite Abschnitt (17b) des zweiten Rastelements (15, 17) oder der Aufnahmeabschnitt andererseits in einem Nichtverbindungszustand unter einem ersten Abstand derart zueinander angeordnet sind, dass der erste Abstand beim Vorgang des Verbindens des Steckkonnektors (100) mit einem Konnektionsabschnitt (300) in einen zweiten Abstand übergeht, welcher größer als der erste Abstand ist.

4. Steckkonnektor (100) nach einem der vorangegangenen Ansprüche, wobei der erste Abschnitt (15b) des ersten Rastelements (15) und/oder der zweite Abschnitt (17b) des zweiten Rastelements (15, 17) als kippbarer Abschnitt ausgestaltet ist.

5. Steckkonnektor (100) nach einem der vorangegangenen Ansprüche, wobei ein Innendurchmesser des Fluidführungsabschnitts (1) des Grundkörpers, welcher sich insbesondere in Längsrichtung des Grundkörpers erstreckt, konstant und vorzugsweise zwischen 4,0 und 4,5 mm, besonders bevorzugt 4,2 mm beträgt.

6. Steckkonnektor (100) nach einem der vorangegangenen Ansprüche, wobei der Grundkörper zusätzlich zum ersten Endbereich (2) und zum zweiten Endbereich (3) des Fluidführungsabschnitts (1) wenigstens eine weitere Öffnung (21) aufweist, welche mittels Septum (23) verschließbar ist.

7. Steckkonnektor (100) nach einem der vorangegangenen Ansprüche, wobei der Grundkörper den Fluidführungsabschnitt (1) als einen Hauptkanal mit einem Lumen zum Leiten eines ersten Fluids durch den Steckkonnektor (100) hindurch aufweist, mit einer Nebenkanalmündung eines Nebenkanals zum Zugeben eines zweiten Fluids in den Hauptkanal,
wobei der Steckkonnektor (100) wenigstens ein relativ zum Grundkörper aus einer ersten Position in eine zweite Position überführbar angeordnetes Betätigungselement (40) aufweist,
wobei der Steckkonnektor (100) einen Dichtabschnitt aufweist, welcher angeordnet ist, um bei Überführen des Betätigungselements (40) von einer Position in die andere zwischen einer ersten Stellung des Dichtabschnitts, in welcher der Dichtabschnitt die Nebenkanalmündung nicht verschließt oder bedeckt (offene Stellung) und einer zweiten Stellung des Dichtabschnitts, in welcher der Dichtabschnitt die Nebenkanalmündung verschließt oder bedeckt (geschlossene Stellung) verdrehbar zu sein.

8. Set, aufweisend, oder bestehend aus, wenigstens einem Steckkonnektor (100) gemäß einem der vorangegangenen Ansprüche, und wenigstens einem Konnektionsabschnitt (300), wobei Steckkonnektor (100) und Konnektionsabschnitt (300) ausgestaltet und vorgesehen sind, um miteinander in Rastverbindung verrastet zu werden.

9. Set nach Anspruch 8, wobei der Innendurchmesser des Fluidführungsabschnitts (1) und ein Innendurchmesser eines Fluidrohrs (301) des Konnektionsabschnitts (300) im Bereich einer dem Fluidführungsabschnitt (1) zugeordneten oder zugewandten Stirnseite gleich sind.

10. Set nach Anspruch 8 oder 9, wobei der Innendurchmesser des Fluidführungsabschnitts (1), ein Innendurchmesser des Dichtelements (200) und ein Innendurchmesser eines Fluidrohrs (301) im Bereich einer dem Fluidführungsabschnitt (1) zugeordneten oder zugewandten Stirnseite gleich sind.

11. Blutbehandlungsvorrichtung (500) oder Verbindungsadapter mit wenigstens einem Konnektionsabschnitt (300), wobei der Konnektionsabschnitt (300) ausgestaltet und vorgesehen ist, um mit wenigstens einem Steckkonnektor (100) gemäß einem der Ansprüche 1 bis 8 in Rastverbindung verrastet zu werden, oder mit wenigstens einem Set gemäß einem der Ansprüche 8 bis 10.

12. Schlauchleitung (30) für medizinische Verwendung, welche wenigstens einen Steckkonnektor (100) gemäß einem der Ansprüche 1 bis 7 aufweist.

13. Schlauchleitung nach Anspruch 12, wobei die Länge des Schlauchs maximal 100 mm beträgt.

14. Blutbehandlungsmaschine, welche mit einer Blutbehandlungsvorrichtung (500) oder einem Verbindungsadapter gemäß Anspruch 11 in Fluidkommunikation verbunden ist.

## Claims

1. A medical plug connector (100) to be plugged on a connection section (300), of for example a blood treatment apparatus (500), or to be plugged on a connection section (300) of a connecting adapter,
wherein the plug connector (100) comprises at least one main body with a receiving section for receiving one end of a fluid pipe (301) of the connection section (300), the main body comprising:
- a first latching element (15) having a first section (15b), wherein the first section (15b) is designed to be elastically bendable or elastically tiltable;
- optionally, a second latching element (17) having a second section (17b);
- wherein at least one of the elements of a group, which consists of the first latching element (15) and the second latching element (17), comprises a recess (15a) for receiving a protrusion (305a) of the connection section (300) and/or an elevation to reach into a recess, for example of the blood treatment apparatus (500), or of the connecting adapter, wherein the recess (15a, 17a) or the elevation of a latching element (15, 17) from the group faces the other latching element (17, 15) from the group respectively and/or the receiving section, or is arranged on an inner side of the respective latching element (15, 17),
wherein the plug connector comprises a fluid guiding section (1) in its main body, through which fluid can flow through the plug connector (100) in a longitudinal direction therefrom,
wherein the main body consists of a first material, or comprises such a material, wherein the plug connector (100) comprises at least one sealing element (200) made of a second material different from the first material,
**characterized in that**
the sealing element (200) comprises a through opening with at least one inner diameter, which corresponds to an inner diameter of the fluid guiding section (1) of the main body.

2. The plug connector (100) according to claim 1, wherein the second section (17b) of the second latching element (17) is also designed to be elastically bendable or elastically tiltable.

3. The plug connector (100) according to anyone of the preceding claims, wherein the first section (15b) of the first latching element (15) on the one side and the second section (17b) of the second latching element (15, 17) or the receiving section on the other side, are, in a non-connected state, arranged having a first distance between each other in such a way that, during the process of connecting the plug connector (100) to a connection section (300), the first distance passes into a second distance greater than the first distance.

4. The plug connector (100) according to anyone of the preceding claims, wherein the first section (15b) of the first latching element (15) and/or the second section (17b) of the second latching element (15, 17) is/are designed as a tiltable section.

5. The plug connector (100) according to anyone of the preceding claims, wherein an inner diameter of the fluid guiding section (1) of the main body, which extends in particular in the longitudinal direction of the main body, is constant and preferably between 4.0 and 4.5 mm, particularly preferred is 4.2 mm.

6. The plug connector (100) according to anyone of the preceding claims, wherein the main body comprises, in addition to the first end area (2) and to the second end area (3) of the fluid guiding section (1), at least one further opening (21) which is closable by means of a septum (23).

7. The plug connector (100) according to anyone of the preceding claims, wherein the main body comprises the fluid guiding section (1) as a main canal with a lumen for guiding a first fluid through the plug connector (100), with a secondary canal outlet of a secondary canal for adding a second fluid into the main canal,
wherein the plug connector (100)comprises at least one actuating element (40) which is arranged relative to the main body to be transferrable from a first position into a second position,
wherein the plug connector (100) comprises a sealing section, which is arranged to be twistable, when the actuating element (40) is being transferred from one position into the other, between a first position of the sealing section in which the sealing section does not close or cover the secondary canal outlet (open position) and a second position of the sealing section in which the sealing section closes or covers the secondary canal outlet (closed position) .

8. A set, comprising, or consisting of, at least one plug connector (100) according to anyone of the preceding claims, and at least a connection section (300), wherein the plug connector (100) and the connection section (300) are designed and provided to be latched together in a latching connection.

9. The set according to claim 8, wherein the inner diameter of the fluid guiding section (1) and an inner diameter of a fluid pipe (301) of the connection section (300) are the same in an area of a front face assigned to, or facing the fluid guiding section (1).

10. The set according to claim 8 or 9, wherein the inner diameter of the fluid guiding section (1), an inner diameter of the sealing element (200) and an inner diameter of a fluid pipe (301) are the same in an area of a front face assigned to, or facing the fluid guiding section (1).

11. A blood treatment apparatus (500) or a connection adapter comprising at least one connection section (300), wherein the connection section (300) is designed and provided to be latched in a latching connection with at least one plug connector (100) according to anyone of claims 1 to 7 or with at least one set according to anyone of claims 8 to 10.

12. A tube line (30) for medical use comprising at least one plug connector (100) according to anyone of claims 1 to 7.

13. The tube line according to claim 12, wherein the length of the tube is 100 mm maximally.

14. A blood treatment machine which is connected in fluid communication with a blood treatment apparatus (500) or with a connecting adapter according to claim 11.

## Revendications

1. Un connecteur médical enfichable (100) à enficher sur une section de raccordement (300), par exemple d'un appareil de traitement du sang (500), ou à enficher sur une section de raccordement (300) d'un adaptateur de raccordement,
où le connecteur enfichable (100) comprend au moins un corps de base avec une section de réception pour recevoir une extrémité d'un conduit de fluide (301) de la section de raccordement (300), le corps de base comprenant:
- un premier élément d'enclenchement (15) ayant une première partie (15b), la première partie (15b) étant conçue pour être élastiquement pliable ou élastiquement inclinable;
- optionnellement, un second élément d'enclenchement (17) ayant une seconde partie (17b);
- où au moins l'un des éléments d'un groupe, constitué du premier élément d'enclenchement (15) et du second élément d'enclenchement (17), présente un évidement (15a) destiné à recevoir une saillie (305a) de la section de raccordement (300) et/ou une élévation pour s'engager dans un évidement, par exemple de l'appareil de traitement du sang (500) ou de l'adaptateur de raccordement,
où l'évidement (15a, 17a) ou l'élévation d'un élément d'enclenchement (15, 17) du groupe fait face respectivement à l'autre élément d'enclenchement (17, 15) du groupe et/ou à la section de réception, ou est agencé sur une face interne de l'autre élément d'enclenchement (15, 17) respectivement,
où le connecteur enfichable comprend une section de guidage de fluide (1) dans son corps de base, au travers de laquelle un fluide peut s'écouler via le connecteur enfichable (100) dans une direction longitudinale de celui-ci,
où le corps de base est constitué d'un premier matériau, ou en en comprend un tel, où le connecteur enfichable (100) comprend au moins un élément d'étanchéité (200) fait d'un second matériau différent du premier matériau,
**caractérisé en ce que**
l'élément d'étanchéité (200) comprend une ouverture de passage avec au moins un diamètre intérieur correspondant à un diamètre intérieur de la section de guidage de fluide (1) du corps de base.

2. Le connecteur enfichable (100) selon la première revendication, où la seconde partie (17b) du second élément d'enclenchement (17) est également conçue pour être élastiquement pliable ou élastiquement inclinable.

3. Le connecteur enfichable (100) selon l'une quelconque des revendications précédentes, où la première partie (15b) du premier élément d'enclenchement (15), d'une part, et la seconde partie (17b) du second élément d'enclenchement (15, 17) ou la section de réception, d'autre part, sont agencées, dans un état de non-raccordement, à une première distance l'une de l'autre, de sorte que, pendant le processus de raccordement du connecteur enfichable (100) à une section de raccordement (300), la première distance passe à une seconde distance supérieure à la première distance.

4. Le connecteur enfichable (100) selon l'une quelconque des revendications précédentes, où la première partie (15b) du premier élément d'enclenchement (15) et/ou la seconde partie (17b) du second élément d'enclenchement (15, 17) est/sont conçue(s) sous la forme d'une partie inclinable.

5. Le connecteur enfichable (100) selon l'une quelconque des revendications précédentes, où un diamètre intérieur de la section de guidage de fluide (1) du corps de base, qui s'étend notamment dans la direction longitudinale du corps de base, est constant et est compris de préférence entre 4,0 et 4,5 mm, plus particulièrement est de 4,2 mm.

6. Le connecteur enfichable (100) selon l'une quelconque des revendications précédentes, où le corps de base comprend, en plus de la première extrémité (2) et de la seconde extrémité (3) de la section de guidage de fluide (1), au moins une ouverture supplémentaire (21) pouvant être fermée au moyen d'un septum (23).

7. Le connecteur enfichable (100) selon l'une quelconque des revendications précédentes, où le corps de base comprend la section de guidage de fluide (1) en tant que canal principal avec une lumière pour guider un premier fluide au travers du connecteur enfichable (100), avec une embouchure de canal secondaire d'un canal secondaire pour ajouter un second fluide dans le canal principal,
où le connecteur enfichable (100) comprend au moins un élément d'actionnement (40) agencé par rapport au corps de base de façon à pouvoir être transféré d'une première position à une seconde position,
où le connecteur enfichable (100) comprend une section d'étanchéité agencée pour, lorsque l'élément d'actionnement (40) est transféré d'une position à l'autre, pouvoir basculer entre une première position de la section d'étanchéité dans laquelle la section d'étanchéité ne ferme pas ou ne recouvre pas l'embouchure de canal secondaire (position ouverte) et une seconde position de la section d'étanchéité dans laquelle la section d'étanchéité ferme ou recouvre l'embouchure de canal secondaire (position fermée).

8. Un set, comprenant ou composé d'au moins un connecteur enfichable (100) selon l'une quelconque des revendications précédentes, et d'au moins une section de raccordement (300), où le connecteur enfichable (100) et la section de raccordement (300) sont conçus et prévus pour s'enclencher ensemble par encliquetage.

9. Le set selon la revendication 8, où le diamètre intérieur de la section de guidage de fluide (1) et un diamètre intérieur d'un conduit de fluide (301) de la section de raccordement (300) sont identiques dans la zone d'une face frontale affectée ou faisant face à la section de guidage de fluide (1).

10. Le set selon la revendication 8 ou 9, où un diamètre intérieur de la section de guidage de fluide (1), un diamètre intérieur d'un élément d'étanchéité (200) et un diamètre intérieur d'un conduit de fluide (301) sont identiques dans la zone d'une face frontale affectée ou faisant face à la section de guidage de fluide (1).

11. Un appareil de traitement du sang (500) ou un adaptateur de raccordement comprenant au moins une section de raccordement (300), où la section de raccordement (300) est conçue et prévue pour s'enclencher par encliquetage avec au moins un connecteur enfichable (100) selon l'une quelconque des revendications 1 à 7 ou avec au moins un set selon l'une quelconque des revendications 8 à 10.

12. Une tuyauterie (30) à usage médical comprenant au moins un connecteur enfichable (100) selon l'une quelconque des revendications 1 à 7.

13. La tuyauterie selon la revendication 12, où la longueur du tuyau est d'au maximum 100 mm.

14. Une machine de traitement du sang reliée en communication fluidique à un appareil de traitement du sang (500) ou à un adaptateur de raccordement selon la revendication 11.
